# EUROPEAN PATENT SPECIFICATION

(11) **EP 2 632 489 B1**
(45) Date of publication and mention of the grant of the patent: **15.01.2020**
(21) Application number: 11837082.4
(22) Date of filing: 27.10.2011
(51) Int. Cl.: A61P 35/00, A61K 39/00, C07K 16/28, A61K 45/00, C07K 14/47, G01N 33/50, A61K 39/395, A61K 45/06, A61K 51/10, G01N 33/574

(54) **COMPOSITIONS TARGETING THE SOLUBLE EXTRACELLULAR DOMAIN OF E-CADHERIN AND RELATED METHODS FOR CANCER THERAPY**
ZUSAMMENSETZUNGEN ZUR ANZIELUNG DER LÖSLICHEN EXTRAZELLULÄREN DOMÄNE VON E-CADHERIN SOWIE ZUGEHÖRIGE KREBSTHERAPIEVERFAHREN
COMPOSITIONS CIBLANT LE DOMAINE EXTRACELLULAIRE SOLUBLE DE L'E-CADHÉRINE ET MÉTHODES DE THÉRAPIE ANTICANCÉREUSE APPARENTÉES

(30) Priority: 27.10.2010 US 407367 P
(43) Date of publication of application: 04.09.2013
(73) Proprietor: The Research Foundation for The State University of New York, Albany NY 12207-2826 (US)
(72) Inventor: BROUXHON, Sabine, East Setauket, NY 11733 (US); O'BANION, M. Kerry, Pittsford, NY 14534 (US); KYRKANIDES, Stephanos, Stony Brook, NY 11790 (US)
(74) Representative: Chapman, Paul Gilmour
(86) International application number: PCT/US2011/058076
(87) International publication number: WO 2012/058418

(56) References cited:
- WO-A1-94/11401
- WO-A2-02/34880
- SHIRAISHI K, TSUZAKA K, YOSHIMOTO K, KUMAZAWA C, NOZAKI K, ABE T, TSUBOTA K, TAKEUCHI T.: "Critical role of the fifth domain of E-cadherin for heterophilic adhesion with alpha E beta 7, but not for homophilic adhesion", THE JOURNAL OF IMMUNOLOGY, vol. 175, 2005, pages 1014-1021, XP002721706,
- XIN ZHANG ET AL.: 'HOXA1 is required for E-cadherin-dependent anchorage- independent survival of human mammary carcinoma cells' THE JOURNAL OF BIOLOGICAL CHEMISTRY vol. 281, no. 10, 2006, pages 6471 - 6481, XP055105407
- ELAINE M. CORPS ET AL.: 'Role of the a domain in ligand binding by integrin alpha E beta7' EUR. J. IMMUNOL. vol. 33, no. 9, 2003, pages 2599 - 2608, XP055105410
- ALLISON M. FANNON ET AL.: 'Novel E-cadherin-mediated adhesion in peripheral nerve: Schwann cell architecture is stabilized by autotypic adherens junctions' THE JOURNAL OF CELL BIOLOGY vol. 129, no. 1, 1995, pages 189 - 202, XP055105411
- SERGIO GALAZ ET AL.: 'Loss of E-cadherin mediated cell-cell adhesion as an early trigger of apoptosis induced by photodynamic treatment' JOURNAL OF CELLULAR PHYSIOLOGY vol. 205, no. 1, 2005, pages 86 - 96, XP055105412
- J. J. PELUSO ET AL.: 'E-cadherin-mediated cell contact prevents apoptosis of spontaneously immortalized granulosa cells by regulating Akt kinase activity' BIOLOGY OF REPRODUCTION vol. 64, no. 4, 2001, pages 1183 - 1190, XP055105418
- STEPHANE FOUQUET ET AL.: 'Early loss of E-cadherin from cell-cell contacts is involved in the onset of anoikis in enterocytes' THE JOURNAL OF BIOLOGICAL CHEMISTRY vol. 279, no. 41, 2004, pages 43061 - 43069, XP055105416
- Elaine m Corps ET AL: "Role of the[alpha]I domain in ligand binding by integrin[alpha]E[beta]7", European Journal of Immunology, 1 September 2003 (2003-09-01), pages 2599-2608, XP055105410, DOI: 10.1002/eji.200324156 Retrieved from the Internet: URL:http://onlinelibrary.wiley.com/store/1 0.1002/eji.200324156/asset/2599_ftp.pdf?v= 1&t=hsd8c7tn&s=748a2cad50b7e310e715ad02fa1 c9f1ddc3d436c [retrieved on 2016-01-30]

## Description

### STATEMENT REGARDING FEDERALLY FUNDED RESEARCH

This invention was made with government support awarded by the National Institutes of Health under Grant Nos. CA133910 and ES015832. The U.S. government has certain rights in this invention.

### FIELD OF THE INVENTION

The compositions and methods of the present invention are related to targeting the extracellular domain of the cell-cell adhesion protein E-cadherin. The compositions include binding agents, such as antibodies, as well as antigenic fragments of the E-cadherin extracellular domain that can be used in therapeutic and prophylactic methods of treating cancer.

### BACKGROUND

E-cadherin is an integral transmembrane glycoprotein that helps maintain epithelial cell-cell adhesion. Loss of E-cadherin function (full length) has been demonstrated to result in cellular de-differentiation, proliferation and increased invasiveness in cancers of the skin, lung, stomach, intestine and breast (Brouxhon et al., Cancer Res. 67(16):7654-7664 (2007); Hirohashi, Am. J. Pathol. 153(2):333-339 (1998); Chen et al., Cancer Lett. 201:97-106 (2003)). Moreover, loss of E-cadherin staining in biopsy specimens from breast cancer patients has been associated with a poor prognosis and short metastasis-free periods (Pederson et al., Brit. J. Cancer 87:1281-1286 (2002)).

The full length protein is composed of an extracellular domain consisting of five subdomains designated EC1-EC5, a single transmembrane region, and a cytoplasmic domain *(see* Shiraishi et al., J. Immunol. 175(2):1014-1021 (2005)). The EC1 subdomain, which is the most distant from the cell membrane surface, contains a histidine-alanine-valine (HAV) triplet found in cadherin-expressing cells (including E-, N-, P-, and R-cadherin-expressing cells), and this subdomain is thought to be essential for promoting the cell-cell contact mediated by E-cadherin (Beavon, European J. Cancer 36:1607-1620 (2000)). WO 94/11401 A1 (Univ Yale) discloses antibodies which target EC1.

Full-length E-cadherin contains a cleavage site for various proteases near the transmembrane domain, and cleavage at that site produces a soluble N-terminal peptide of ∼80-84 kDa called soluble E-cadherin (sEcad). Shedding of sEcad occurs constitutively at low levels in normal, unstimulated epithelial cells and at elevated levels in patients with epithelial-derived tumors such as breast, skin, lung, prostate, gastric and colorectal cancers (Banks et al., J. Clin. Pathol. 48:179-180 (1995); Baranwal et al., Biochem. Biophys. Res. Com. 384(1):6-11 (2009); Chan et al., Gut 48:808-811 (2001); Charalabopoulos et al., Exp. Oncol. 28(1):83-85 (2006); Kuefer et al., Clin. Cancer Res. 9:6447-6452 (2003); Shirahama et al., J. Dermatol. Sci. 13:30-36 (1996); Velikova et al., Br. J. Cancer 77:1857-1863 (1998)). Shedding of sEcad has also been reported to increase in normal, non-cancerous canine kidney cells after the induction of apoptosis (Steinhusen et al., J. Biol. Chem. 276:4972-4980 (2001).

While sEcad levels are increased in the urine or sera of cancer patients, and elevated when normal cells undergo apoptosis, the biologic activity of this shed protein is not well understood. A number of studies have demonstrated that sEcad disrupts normal epithelial cell-cell adhesion, induces epithelial cell scattering, and enhances tumor cell proliferation, migration, and invasion (Gil et al., Gynecol. Oncol. 108(2):361-369 (2008); Maretzky et al., Proc. Natl. Acad. Sci. USA 102(26):9182-9187 (2005); Marambaud et al., EMBO J. 21(8):1948-1956 (2002); Najy et al., J. Biol. Chem. 283(26):18393-18401 (2008); Noe et al., J. Cell Sci. 114:111-118 (2001); Ryniers et al., Biol. Chem. 383:159-165 (2002); and Symowicz et al., Cancer Res. 67(5):2030-2039 (2007)). The signaling pathways modulating these biologic functions are still unclear. Studies using the SKBr3 breast cancer cell line demonstrated that sEcad-HER2 complexes were induced by exogenous addition of a purified extracellular fusion protein (Fc-sEcad), leading to extracellular signal-regulated kinase (ERK) activation (Najy et al., J. Biol. Chem. 283(26):18393-18401 (2008)). The human EGF receptor belongs to the ErbB or HER family of receptor tyrosine kinases, which are overexpressed or dysregulated in many epithelial tumors (Mendelsohn and Baselga, Oncogene 19:6550-6565 (2000); Burgess, Growth Factors 26:263-274 (2008)). This family of receptors activates downstream-signaling molecules such as ERK, which in turn activates a range of cancer cell behaviors including cell proliferation, migration, invasion and angiogenesis (Hanahan and Weinberg, Cell 100:57-70 (2000); Shields et al., Trends Cell Biol. 10:147-154 (2000)). Accordingly, a number of anti-EGF therapies have been developed. These include small molecule tyrosine kinase inhibitors, monoclonal antibodies, and cancer vaccines (Fukuoka et al., Proc. Am. Soc. Clin. Oncol. 21:292a Abs1188 (2002); Lage et al., Ann. Med. 35:327-336 (2003); Mateo et al., Immunotechnology 3:71-81 (1997); Slamon et al., N. Engl. J. Med. 344:783-792 (2001); and Yu et al., J. Clin. Invest. 110:289-294 (2002)). These therapeutic strategies are limited in that only some tumors, at a defined maturation stage, express the specific receptor/antigen and not all tumors with a certain histology and stage overexpress the target receptor/antigen (only 20% to 50% of breast cancers overexpress the EGF receptor). Thus, response rates for these types of drugs remains low (Mendelson and Baselga, Oncogene 19:6550-6565 (2000); Ortega et al., Cancer Control 17(1):7-15 (2010)). In addition, tumors initially responsive to these drugs eventually develop acquired resistance (Jackman et al., Clin. Cancer Res. 12:3908-3914 (2006); Ortega et al., Cancer Control 17(1):7-15 (2010); and Riely et al., Clin. Cancer Res. 12:839-844 (2006)).

### SUMMARY

The present invention is based, in part, on our discovery that targeting epitopes within one or more of the EC2-EC5 subdomains of E-cadherin results in the death of epithelial-derived tumor cells but does not kill normal, healthy epithelial cells or non-epithelial cells, including endothelial cells and fibroblasts, to any appreciable extent (*e.g.,* to any clinically detrimental extent). Accordingly, the compositions described include polypeptides having an amino acid sequence of one or more of the EC2-EC5 subdomains of E-cadherin and biologically active variants thereof; expression vectors and cells for expressing such polypeptides; and agents (*e.g.,* antibodies) that target the EC2-EC5 subdomains. Methods described include methods of identifying and producing polypeptides having an amino acid sequence of one or more of the EC2-EC5 subdomains of E-cadherin or a biologically active variant thereof; methods of generating agents, such as antibodies, that target these polypeptides; and methods of administering such agents or eliciting their production *in vivo* to treat epithelial cancers or reduce the risk of their occurrence or recurrence. For ease of reading, we will not refer to biologically active variants at every opportunity; it is to be understood that where a polypeptide having an amino acid sequence found in one or more of the EC2, EC3, EC4, and EC5 subdomains of a naturally occurring E-cadherin can be made and used as described herein, a biologically active variant of that polypeptide can also be made and used.

The methods in which anti-E-cadherin antibodies are administered encompass dose-specific therapies, and the therapies can be selectively directed toward and cytotoxic for breast, lung, colon, prostate and skin cancers as well as other epithelial cancers and cancers of tissues derived from the ectoderm (*e.g*., the central nervous system, the lens of the eye, cranial and sensory ganglia and nerves, and connective tissue in the head). The therapeutic and prophylactic methods described herein can be carried out in connection with other cytotoxic therapies (*e.g*., chemotherapy, hormone therapy, radiotherapy, and antibody-based therapies (*e.g.,* monoclonal anti-EGF antibody therapy)).
Accordingly, in a first aspect, the present invention provides an antibody that specifically targets one or more of the second, third, fourth, or fifth subdomains (EC2, EC3, EC4 and EC5, respectively) of soluble E-cadherin (sEcad) but not the first subdomain (EC1) of sEcad for use in a method for the treatment of cancer, wherein the antibody is administered in an amount that selectively kills cancer cells but does not kill normal, healthy cells to any clinically detrimental extent.

Also provided is a pharmaceutical formulation comprising the antibody of the first aspect, wherein the pharmaceutical formulation is free of a cytotoxic amount of an excipient.

Embodiments are provided by the dependent claims.

In a related aspect, there are described methods of treating a patient who has cancer by, *inter alia,* administering to the patient a therapeutically effective amount of an agent that specifically targets one or more of the second, third, fourth, or fifth subdomains (EC2, EC3, EC4 and EC5, respectively) of soluble E-cadherin (sEcad) but not the first subdomain (EC1) of sEcad. The agent can specifically target EC4 and/or EC5. Where a single subdomain (*e.g*., EC4 or EC5) is targeted, the agent can bind amino acid residues confined to that domain. The agent can be a protein scaffold, such as an antibody or a fragment or other variant thereof that specifically binds an epitope comprising amino acid residues in one or more of the EC2, EC3, EC4 or EC5 subdomains of sEcad but not in the EC1 subdomain of sEcad.

The antibody can be a humanized, chimeric, murine, or human antibody. The antibody can also be a single chain antibody; the antibody can also be a monoclonal or polyclonal antibody (*e.g*., an immunoglobulin of the IgG or IgM class). Regardless of the precise nature of the agent, it can be detectably labeled (*e.g*., with a fluorescent or chemiluminescent tag).

With regard to impact on biological cells, the agent can be characterized as one that kills malignant E-cadherin-expressing cells but does not kill non-malignant cells to any significant or appreciable extent. The killing can be achieved by inducing programmed cell death, growth arrest, anoikis, necrosis, autophagy, or another state that results in cell death.

The agents described can be formulated as pharmaceutical formulations or preparations that are free of cytotoxic amounts of an excipient or other "inert" ingredient. More specifically, the pharmaceutical or pharmaceutically acceptable compositions can be formulated for delivery to a patient by oral administration, intravenous administration, nasal or inhalation administration (*e.g*., insufflation), intramuscular administration, intraperitoneal administration, transmucosal administration (*e.g.,* formulated for administration to mucosal tissue such as that lining the rectum or vagina), or transdermal administration. While we discuss dosages further below, we note here that the agent can be delivered in a pharmaceutical formulation containing about 1 µg/mL to about 400 µg/mL of the agent (*e.g*., about 4 µg/mL to about 20 µg/mL of the agent). The dosage can also be such that, upon administration to a patient, the patient's serum level of the active pharmaceutical agent is about 1-10 mg/kg (*e.g*., about 1-5 mg/kg). When used in cell culture, the dosage can be such that, upon addition to culture medium, the active pharmaceutical agent is present at about 1-500 µg/mL of cell culture medium (*e.g*., about 1-400 µg/mL). As is recognized in the art, dosages can vary in different formulations, and the dosages within the present pharmaceutical formulations or preparations can vary depending on the presence, absence, or relative amount of additives in the formulation (*e.g*., as supplied by a manufacturer). Thus, the methods described encompass those in which the agent is delivered in a pharmaceutical formulation that: (a) produces, upon administration to a patient, a serum level of the agent of about 1-10 mg/kg (*e.g*., about 1-5 mg/kg), or (b) produces, upon addition to a cell culture, a concentration of the agent of about 1-500 µg/mL (1-400 µg/mL) of cell culture medium.

In any of the methods described in which an sEcad-targeting agent is administered, the method can include a step in which one provides a biological sample from the patient (*e.g*., prior to administering the targeting agent and/or at some point in time after administering the targeting agent) and determines whether the sample includes an elevated level of sEcad or another predictive biomarker for cancer. The biological sample can be, for example, a urine, saliva, cerebrospinal fluid, blood, or biopsy sample. Where such a step is carried out before administering the agent, an elevated level of sEcad can indicate that the patient is a good candidate for the treatment. Where such a step is carried out at one or more times after administering the agent, a reduced level of sEcad can indicate that the patient is responding well to the treatment.

In any of the methods described, one can also administer a second cancer treatment. For example, one can administer a chemotherapeutic agent, a radiation treatment, a treatment with an antibody (*i.e.,* an antibody that is useful in the treatment of a cancer and specifically binds a target other than sEcad), or surgical intervention.

Patients amenable to treatment include those having a cancer within an epithelialized tissue. The cancer can be a cancer of the alimentary canal (*e.g.,* the mouth, throat, esophagus, stomach, intestine, rectum or anus), central nervous system, breast, skin (*e.g.,* a squamous cell carcinoma or melanoma), reproductive system (*e.g*., cervical cancer, uterine cancer, ovarian cancer, vulval or labial cancer, prostate cancer, testicular cancer, or cancer of the male genital tract), lung, or urinary tract.

The methods can be therapeutic or prophylactic. Accordingly, in another related aspect, there are described methods of reducing the likelihood that a subject will develop cancer. These methods can be carried out by administering to the subject a therapeutically effective amount of (a) an antigenic polypeptide that comprises an amino acid sequence from one or more of the EC2-EC5 subdomains of sEcad but excludes the EC1 subdomain, or an antigenically active fragment or other variant thereof or (b) an expression vector comprising a nucleic acid sequence encoding the antigenic polypeptide or the antigenically active fragment or other variant thereof. The antigenic polypeptide can include an amino acid sequence that is confined within EC4, confined within EC5, or that encompasses sequence from both EC4 and EC5. The antigenic polypeptide can elicit the production of antibodies that specifically bind sEcad but do not bind E-cadherin expressed by non-malignant cells in the subject. The antigenic polypeptides and expression vectors employed in these methods can be formulated in ways that are the same as or similar to the formulations described above. For example, they can be delivered by oral administration, intravenous administration (which may be the preferable to oral administration), nasal or inhalation administration (*e.g*., insufflation), intramuscular administration, intraperitoneal administration, transmucosal administration, or transdermal administration. The risks faced by the subject may be a risk of developing any of the types of cancer described herein. The risk may be an average risk based on the general rate of occurrence in the population, or it may be an enhanced risk due to a genetic predisposition or an earlier occurrence of the cancer. For example, antigenic polypeptides or vectors encoding them can be administered to a subject to reduce the risk of an occurrence or recurrence of a cancer within an epithelialized tissue; a cancer of the alimentary canal (*e.g.,* the mouth, throat, esophagus, stomach, intestine, rectum or anus), central nervous system, breast, skin (*e.g.,* a squamous cell carcinoma or melanoma), reproductive system (*e.g*., cervical cancer, uterine cancer, ovarian cancer, vulval or labial cancer, prostate cancer, testicular cancer, or cancer of the male genital tract), lung, or urinary tract.

The methods described can be expressed in terms of the preparation of a medicament. Accordingly, the invention relates to the use of the agents and compositions described herein in the preparation of a medicament. Use of the agents and compositions extends to the preparation of a medicament for the treatment of cancer (including the types of cancer described herein).

The compositions of the invention include pharmaceutically acceptable compositions that include a therapeutically effective amount of (a) an antibody that specifically binds an epitope comprising amino acid residues in one or more of the EC2, EC3, EC4 or EC5 subdomains of sEcad but not in the EC1 subdomain of sEcad. Compositions may include(b) an antigenic polypeptide that comprises an amino acid sequence from one or more of the EC2-EC5 subdomains of sEcad but excludes the EC1 subdomain, or an antigenically active fragment or other variant thereof; or (c) an expression vector comprising a nucleic acid sequence encoding the antigenic polypeptide or the antigenically active fragment or other variant thereof.

In yet another related aspect, the present invention features methods for identifying an epitope in an sEcad. These methods can be carried out by, *inter alia:* (a) providing a polypeptide comprising sEcad or a fragment or other variant thereof; (b) administering the polypeptide to an animal; (c) isolating antibodies produced by the animal in response to the polypeptide; and (d) exposing cancerous cells to the antibodies or to a monoclonal antibody generated therefrom. The death of the cancerous cells indicates that the polypeptide comprises an epitope of sEcad that can be targeted or used as an agent in cancer treatment, prophylaxis, or imaging. As previously described, the polypeptide can include an amino acid sequence from one or more of the EC2, EC3, EC4, or EC5 subdomains of sEcad but exclude amino acid sequence from the EC1 subdomain of sEcad. For example, the polypeptide can include an amino acid sequence from one or more of EC4 and/or EC5 (*e.g.,* an amino acid sequence confined to EC4 or an amino acid sequence confined to EC5).

One of the greatest challenges in developing cancer treatments lies in finding therapeutic agents that can distinguish between cancerous cells and normal healthy tissues. Many of the currently available chemotherapeutics are non-selectively cytotoxic and have a narrow therapeutic index, resulting in systemic drug toxicity that is debilitating to patients and heightens overall patient mortality. Thus, a highly desirable attribute of new cancer therapeutics is an ability to selectively target cancer cells without deleteriously affecting normal healthy cells and tissues. While the compositions of the present invention are not limited to those that impact cells at any particular point in a signaling pathway, our expectation is that the therapeutic compositions of the present invention will act upstream of the HER2 receptor and will capture a wider array of downstream targets than HER2-targeted treatments.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

FIG. 1A shows the amino acid sequence of human E-cadherin (SEQ ID NO:1), with the extracellular subdomains EC2-EC5 indicated by alternating underlining (EC2 and EC4 are underlined with double lines, and EC3 and EC5 are underlined with single lines).
FIG. 1B is a schematic representation of a wild type human E-cadherin with sEcad as indicated. The length of the soluble fragment can vary and may terminate in the 4^{th} or 5^{th} extracellular domain or, in other cases, in the transmembrane domain (*see,* for example, the study by Noe et al., J. Cell Sci. 114(1):111-118, 2000).
FIG. 2A is a line graph depicting the number of palpable tumors in mice, over time, following treatment with saline or α-sEcad as described in Example 5.
FIG. 2B is a panel of photographs showing, on the left, the tumors visible in a saline-treated mouse versus those in an α-sEcad treated mouse (as described in Example 5). Surgically removed tumors are also shown, as are histological preparations of tissue from untreated (saline) and treated (a-sEcad) mice.
FIG. 2C is a pair of bar graphs illustrating the difference in tumor weight (g) and volume (cm³) in untreated (saline) and treated (a-sEcad) mice.

### DETAILED DESCRIPTION

As described further below, we tested the effectiveness of a variety of commercially available monoclonal and polyclonal antibodies targeting the extracellular domain of E-cadherin, including the DECMA-1 antibody employed by Espada et al. (J. Cell Physiol. 219:84-93 (2009)), Fouquet et al. (J. Biol. Chem. 279(41):43061-43069 (2004)) and Galaz et al. (J. Cell Physiol. 205(1):86-96 (2005)) in a panel of both epithelial cancer cells and non-cancerous cells. When we repeated these experiments, we found that application of this antibody at concentrations as low as 40 µg/mL surprisingly induced cell death in both cancer cells (*i.e.,* MCF-7, SCC12b, SCC13, CRL-1555, PAM212, SP308 and KLN205 cells) as well as in non-cancerous cells that were employed as controls (*i.e*., human breast epithelial cells and PHK and PMK cells). Moreover, treatment of these cancer and non-cancerous cells with the control IgG isotype antibody at the same concentrations (40 µg/mL) also induced the same level of cell death in both types of cells. These data suggest a non-specific induction of cell death after the application of the antibody. In our laboratory, applying a more dilute solution of the antibody targeting the extracellular domains EC2-EC5 of E-cadherin (a low dose of 10-20 µg/mL) induced cell death, apparently by apoptosis, in cancer cells only. We observed no untoward effects on non-cancerous cells. Moreover, application of the control IgG isotype to non-cancerous cells at a concentration of 10-20 µg/mL had no detectable effect on cell viability in general. These concentrations between 10-20 µg/mL are ∼20-50 times lower than the concentration used by Espada et al. (J. Cell Physiol. 219:84-93 (2009)), Fouquet et al. (J. Biol. Chem. 279(41):43061-43069 (2004)) and Galaz et al. (J. Cell Physiol. 205(1):86-96 (2005)). Accordingly, it is our expectation that the present pharmaceutical formulations and preparations can be made and used as low dose formulations (*e.g*., at doses lower than those suggested by Espada and others in prior studies).

As described above, exogenous application of 10-20 µg/mL of antibodies (monoclonal or polyclonal) against the EC2-EC5 subdomains of E-cadherin selectively killed a representative panel of human and mouse tumor cell lines. We also have data showing that such antibodies are cytotoxic to the HT29 human colon cell line, the NCI-H292 human lung cell line, and the KLN205 murine lung cancer cell line. Moreover, we demonstrated that non-cancerous cells, including normal human breast epithelial cells, normal human and mouse keratinocytes, mouse 3T3 fibroblasts and human endothelial cells remained unaffected in our analyses using antibodies that target the subdomains at low concentrations. The molecular pathways by which targeting varying combinations of the EC2-EC5 extracellular domains of E-cadherin in epithelial-derived tumor cells induces cell death has yet to be elucidated. However, we demonstrated that dying cancer cells upregulated the pro-apoptotic marker p53; we observed this upregulation in the MCF-7 breast cancer, mouse SCC and mouse KLN205 lung cancer cell lines after treatment with antibody against the EC2-EC5 E-cadherin domains.

As noted above, the compositions of the invention include antibodies that specifically target one or more of EC2, EC3, EC4 and EC5 of sEcad (but not EC1). These antibodies may subsequently inhibit sEcad, or they may bind, inhibit, or sequester another target, such as a cell survival receptor, in the tumor cell microenvironment. While the present compositions are not limited to those that exert their effect by any particular mechanism, our working hypothesis is the antibodies of the invention interfere with the ability of sEcad to provide signals beneficial to cancer cells. For example, we hypothesize that cancer cells secrete sEcad into the micorenvironment where it provides a functional scaffold that mimics normal cell-to-cell contact. Thus, actually or effectively removing sEcad from the tumor microenvironment perturbs the ability of tumor cells to remain adherent and survive. In other instances, an antibody of the invention may inhibit sEcad activity by binding to an epitope on sEcad that interacts with another cellular target (*e.g*., HER-2), thereby altering downstream signaling events involved in cell survival, cell proliferation, cell migration and/or invasion. Alternatively, or in addition, an antibody may not bind a specific epitope required for sEcad signalling but may instead bind sEcad in a way that sequesters, tags, or targets it for destruction, thereby lowering its concentration in the tumor microenvironment and rendering it unavailable to mimic cell-cell interactions or binding to cellular receptors. For example, the antibodies and compositions may reduce sEcad shedding by, for example, binding to E-cadherin and blocking the cleavage site or otherwise blocking or inhibiting the release of sEcad. Thus, a composition or antibody , as described herein, can specifically target one or more of the EC2-EC5 subdomains, effectively preventing sEcad from providing one or more of the signals it otherwise would by interfering with a specific epitope or actually reducing sEcad levels.

For ease of reading, we may refer to an agent that specifically targets amino acid residues in one or more of the EC2-EC5 subdomains of sEcad but not in the first subdomain (EC1) of sEcad more simply as a targeting agent. The targeting agents can be a protein scaffold, such as a modified fibronectin domain or an immunoglobulin, or a fragment or other variant thereof, that specifically binds to amino acid residues in one or more of the EC2-EC5 subdomains of sEcad (but not to EC1 of sEcad). Where the agent is, or includes, a protein (*e.g.,* a protein scaffold or antigenic polypeptide), we may refer to the agent as a protein-based therapeutic. We tend to use the term "protein" to refer to longer amino acid polymers, and we tend to use the term "polypeptide" to refer to shorter sequences or to a chain of amino acid residues within a larger molecule or complex. Both terms, however, are meant to describe an entity of two or more subunit amino acids, amino acid analogs, or other peptidomimetics, regardless of post-translational modification (*e.g*., amidation, phosphorylation or glycosylation). The subunit amino acid residues can be linked by peptide bonds or other bonds such as, for example, ester or ether bonds. The terms "amino acid" and "amino acid residue" refer to natural and/or non-natural or synthetic amino acids, which may be D- or L-form optical isomers.

The anti-sEcad antibodies can assume various configurations and encompass proteins consisting of one or more polypeptides substantially encoded by immunoglobulin genes. Any one of a variety of antibody structures can be used, including an intact antibody, antibody multimers, or antibody fragments or other variants thereof that include functional, antigen-binding regions of the antibody. We may use the term "immunoglobulin" synonymously with "antibody." The antibodies may be monoclonal or polyclonal in origin. Regardless of the source of the antibody, suitable antibodies include intact antibodies, for example, IgG tetramers having two heavy (H) chains and two light (L) chains, single chain antibodies, chimeric antibodies, humanized antibodies, complementary determining region (CDR)-grafted antibodies as well as antibody fragments, *e.g*., Fab, Fab', F(ab')2, scFv, Fv, and recombinant antibodies derived from such fragments, *e.g*., camelbodies, microantibodies, diabodies and bispecific antibodies.

An intact antibody is one that comprises an antigen-binding variable region (V_{H} and V_{L}) as well as a light chain constant domain (C_{L}) and heavy chain constant domains, C_{H1}, C_{H2} and C_{H3}. The constant domains may be native sequence constant domains (*e.g*. human native sequence constant domains) or amino acid sequence variants thereof. As is well known in the art, the V_{H} and V_{L} regions are further subdivided into regions of hypervariability, termed "complementarity determining regions" (CDRs), interspersed with the more conserved framework regions (FRs). The extent of the FRs and CDRs has been defined (see, Kabat et al. Sequences of Proteins of Immunological Interest, Fifth Edition, U.S. Department of Health and Human Services, NIH Publication No. 91-3242, 1991, and Chothia, et al., J. Mol. Biol. 196:901-917 (1987). The CDR of an antibody typically includes amino acid sequences that together define the binding affinity and specificity of the natural Fv region of a native immunoglobulin binding site.

An anti-sEcad antibody can be from any class of immunoglobulin, for example, IgA, IgG, IgE, IgD, IgM (as well as subtypes thereof (*e.g.,* IgG₁, IgG₂, IgG₃, and IgG₄)), and the light chains of the immunoglobulin may be of types kappa or lambda. The recognized human immunoglobulin genes include the kappa, lambda, alpha (IgA₁ and IgA₂), gamma (IgG₁, IgG₂, IgG₃, IgG₄), delta, epsilon, and mu constant region genes, as well as the myriad immunoglobulin variable region genes.

The term "antigen-binding portion" of an immunoglobulin or antibody refers generally to a portion of an immunoglobulin that specifically binds to a target, in this case, an epitope comprising amino acid residues within or between one or more of the second to fifth subdomains of sEcad (*e.g*., within or between the fourth and fifth subdomains). An antigen-binding portion of an immunoglobulin is therefore a molecule in which one or more immunoglobulin chains are not full length, but which specifically binds to a cellular target. Examples of antigen-binding portions or fragments include: (i) an Fab fragment, a monovalent fragment consisting of the VLC, VHC, CL and CH1 domains; (ii) a F(ab')₂ fragment, a bivalent fragment comprising two Fab fragments linked by a disulfide bridge at the hinge region; (iii) a Fv fragment consisting of the VLC and VHC domains of a single arm of an antibody, and (v) an isolated CDR having sufficient framework to specifically bind, *e.g.,* an antigen binding portion of a variable region. An antigen-binding portion of a light chain variable region and an antigen binding portion of a heavy chain variable region, *e.g.,* the two domains of the Fv fragment, VLC and VHC, can be joined, using recombinant methods, by a synthetic linker that enables them to be made as a single protein chain in which the VLC and VHC regions pair to form monovalent molecules (known as single chain Fv (scFv); see *e.g.,* Bird et al., Science 242:423-426 (1988); and Huston et al., Proc. Natl. Acad. Sci. USA 85:5879-5883 (1988)). Such scFvs can be a target agent of the present invention and are encompassed by the term "antigen-binding portion" of an antibody.

An "Fv" fragment is the minimum antibody fragment that contains a complete antigen-recognition and binding site. This region consists of a dimer of one heavy chain and one light chain variable domain in tight, non-covalent association. It is in this configuration that three hypervariable regions of each variable domain interact to define an antigen-binding site on the surface of the V_{H}-V_{L} dimer. While six hypervariable regions confer antigen-binding specificity, even a single variable domain (or half of an Fv comprising only three hypervariable regions specific for an antigen) has the ability to recognize and bind antigen, although at a lower affinity than the entire binding site. To improve stability, the VH-VL domains may be connected by a flexible peptide linker such as (Gly₄Ser)₃ to form a single chain Fv or scFV antibody fragment or may be engineered to form a disulfide bond by introducing two cysteine residues in the framework regions to yield a disulfide stabilized Fv (dsFv).

As noted, other useful antibody formats include diabodies, minibodies and bispecific antibodies. A diabody is a homodimer of scFvs that are covalently linked by a short peptide linker (about 5 amino acids or less). By using a linker that is too short to allow pairing between two domains on the same chain, the domains can be forced to pair with the complementary domains of another chain and create two antigen-binding sites (*see, e.g.,* EP 404,097 and WO 93/11161 for additional information regarding diabodies). A diabody variant, (dsFv)2 or a linear antibody useful in the present compositions and methods includes a pair of tandem Fd segments (V_{H}-C_{H}1-V_{H}-C_{H}1) that form a pair of antigen binding regions (*see, e.g.,* Zapata *et al., Prot. Eng.* 8:1057 (1995)). Useful minibodies are homodimers of scFv-C_{H3} fusion proteins. In the minibody variant, the Flex minibody, the scFv is fused to the hinge region of IgG1, which is in turn, linked to the CH₃ region by a 10-amino acid linker.

A bispecific antibody, which recognizes two different epitopes, can also be used as long as one arm specifically binds sEcad as described herein. A variety of different bispecific antibody formats have been developed. For example, useful bispecific antibodies can be quadromas, *i.e*., an intact antibody in which each H-L pair is derived from a different antibody. Typically, quadromas are produced by fusion of two different B cell hybridomas, followed by screening of the fused calls to select those that have maintained the expression of both sets of clonotype immunoglobulin genes. Alternatively, a bispecific antibody can be a recombinant antibody. Exemplary formats for bispecific antibodies include, but are not limited to tandem scFvs in which two single chains of different specificity are connected via a peptide linker; diabodies and single chain diabodies.

Fragments of antibodies are suitable for use so long as they retain the desired specificity of the full-length antibody and/or sufficient specificity to inhibit cancer cell survival, proliferation, or metastasis. Thus, a fragment of an anti-sEcad antibody, as described herein, can retain the ability of the intact antibody to bind to the recited subdomains. These antibody portions can be obtained using conventional techniques known to one of ordinary skill in the art, and the portions can be screened for utility in the same manner as intact antibodies are screened as anti-cancer agents.

Methods for preparing antibody fragments are well known in the art and encompass both biochemical methods (e.g. proteolytic digestion of intact antibodies which may be followed by chemical cross-linking) and recombinant DNA-based methods in which immunoglobulin sequences are genetically engineered to direct the synthesis of the desired fragments. Exemplary biochemical methods are described in U.S. Patent Nos. 5,855,866; 5,877,289; 5,965,132; 6,093,399; 6,261,535; and 6,004,555. Nucleic acids encoding a chimeric or humanized chain can be expressed to produce a contiguous polypeptide. See, *e.g.,* Cabilly et al., U.S. Patent No. 4,816,567; Cabilly *et al.,* European Patent No. 0,125,023 B1; Boss et al., U.S. Patent No. 4,816,397; Boss *et al.,* European Patent No. 0,120,694 B1; Neuberger et al., WO 86/01533; Neuberger *et al.,* European Patent No. 0,194,276 B1; Winter, U.S. Patent No. 5,225,539; and Winter, European Patent No. 0,239,400 B1. See also, Newman et al., BioTechnology 10:1455-1460 (1992), regarding CDR-grafted antibodies and Ladner et al. (U.S. Pat. No. 4,946,778) and Bird et al., Science 242:423-426 (1988)) regarding single chain antibodies.

Antibody fragments can be obtained by proteolysis of the whole immunoglobulin by the non-specific thiolprotease, papain. Papain digestion yields two identical antigen-binding fragments, termed "Fab fragments," each with a single antigen-binding site, and a residual "Fc fragment." The various fractions can be separated by protein A-Sepharose or ion exchange chromatography. The usual procedure for preparation of F(ab')₂ fragments from IgG of rabbit and human origin is limited proteolysis by the enzyme pepsin. Pepsin treatment of intact antibodies yields an F(ab')₂ fragment that has two antigen-combining sites and is still capable of cross-linking antigen. A Fab fragment contains the constant domain of the light chain and the first constant domain (CH1) of the heavy chain. Fab' fragments differ from Fab fragments by the addition of a few residues at the carboxyl terminus of the heavy chain CH1 domain including one or more cysteine(s) from the antibody hinge region. F(ab')₂ antibody fragments were originally produced as pairs of Fab' fragments that have hinge cysteines between them. Other chemical couplings of antibody fragments are known.

Also described are methods of making a targeting agent (*e.g.,* an antibody or an antigen-binding fragment or other variant thereof) that targets sEcad by, for example, specifically binding to the second, third, fourth or fifth subdomain of sEcad (or to an epitope including amino acid residues in two or more of these subdomains). For example, variable regions can be constructed using PCR mutagenesis methods to alter DNA sequences encoding an immunoglobulin chain (*e.g*., using methods employed to generate humanized immunoglobulins; see *e.g.,* Kanunan et al., Nucl. Acids Res. 17:5404 (1989); Sato et al., Cancer Research 53:851-856 (1993); Daugherty et al., Nucleic Acids Res. 19(9):2471-2476 (1991); and Lewis and Crowe, Gene 101:297-302 (1991)). Using these or other suitable methods, variants can also be readily produced. For example, cloned variable regions can be mutagenized, and sequences encoding variants with the desired specificity can be selected (*e.g.,* from a phage library; see *e.g.,* Krebber et al., U.S. Pat. No. 5,514,548; and Hoogenboom et al., WO 93/06213).

Other suitable methods of producing or isolating immunoglobulins that specifically recognize a cellular target as described herein include, for example, methods that rely upon immunization of transgenic animals (*e.g*., mice) capable of producing a full repertoire of human antibodies (see *e.g.,* Jakobovits et al., Proc. Natl. Acad. Sci. USA 90:2551-2555 (1993); Jakobovits et al., Nature 362:255-258 (1993); Lonberg et al., U.S. Patent No. 5,545,806; and Surani et al., U.S. Pat. No. 5,545,807).

As is well known in the art, monoclonal antibodies are homogeneous antibodies of identical antigenic specificity produced by a single clone of antibody-producing cells, and polyclonal antibodies generally recognize different epitopes on the same antigen and are produced by more than one clone of antibody producing cells. Each monoclonal antibody is directed against a single determinant on the antigen. The modifier, monoclonal, indicates the character of the antibody as being obtained from a substantially homogeneous population of antibodies, and is not to be construed as requiring production of the antibody by any particular method. For example, the monoclonal antibodies may be made by the hybridoma method first described by Kohler et al., (Nature 256:495 (1975)) or by recombinant DNA methods (see, *e.g.,* U.S. Pat. No. 4,816,567). The monoclonal antibodies may also be isolated from phage antibody libraries using the techniques described in Clackson et al. (Nature 352:624-628 (1991)) and Marks et al., (J. Mol. Biol. 222:581-597 (1991)), for example.

The monoclonal antibodies herein can include chimeric antibodies, *i.e.,* antibodies that typically have a portion of the heavy and/or light chain identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)). Chimeric antibodies of interest include primatized antibodies comprising variable domain antigen-binding sequences derived from a non-human primate (e.g. apes, Old World monkeys, New World monkeys, prosimians) and human constant region sequences.

Various methods for generating monoclonal antibodies (mAbs) are well known in the art. See, *e.g.,* the methods described in U.S. Patent No. 4,196,265.. The most standard monoclonal antibody generation techniques generally begin along the same lines as those for preparing polyclonal antibodies (Antibodies: A Laboratory Manual, Cold Spring Harbor Laboratory Press, Cold Spring Harbor, N.Y. (1988)). Typically, a suitable animal can be immunized with a selected immunogen to stimulate antibody-producing cells. Rodents such as mice and rats are exemplary animals, although rabbits, sheep, frogs, and chickens can also be used. Mice can be particularly useful (*e.g*., BALB/c mice are routinely used and generally give a higher percentage of stable fusions).

Following immunization, somatic cells with the potential for producing the desired antibodies, specifically B lymphocytes (B cells), can be selected for use in MAb generation and fusion with cells of an immortal myeloma cell, generally one of the same species as the animal that was immunized. Myeloma cell lines suited for use in hybridoma-producing fusion procedures typically are non-antibody-producing, have high fusion efficiency, and enzyme deficiencies that render then incapable of growing in certain selective media which support the growth of only the desired fused cells (hybridomas). Any one of a number of myeloma cells can be used, as are known to those of skill in the art. For example, where the immunized animal is a mouse, one can use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one can use R210.RCY3, Y3-Ag 1.2.3, IR983F, 4B210 or one of the above listed mouse cell lines. U-266, GM1500-GRG2, LICR-LON-HMy2 and UC729-6, all can be useful in connection with human cell fusions.

This culturing can provide a population of hybridomas from which specific hybridomas can be selected, followed by serial dilution and cloning into individual antibody producing lines, which can be propagated indefinitely for production of antibody.

Methods for producing monoclonal antibodies can include purification steps. For example, the antibodies can generally can be further purified, for example, using filtration, centrifugation and various chromatographic methods, such as HPLC or affinity chromatography, all of which are techniques well known to one of ordinary skill in the art. These purification techniques each involve fractionation to separate the desired antibody from other components of a mixture. Analytical methods particularly suited to the preparation of antibodies include, for example, protein A-Sepharose and/or protein G-Sepharose chromatography.

The anti-sEcad antibodies of the invention may include CDRs from a human or non-human source. "Humanized" antibodies are generally chimeric or mutant monoclonal antibodies from mouse, rat, hamster, rabbit or other species, bearing human constant and/or variable region domains or specific changes. Techniques for generating a so-called "humanized" antibody are well known to those of skill in the art.

The framework of the immunoglobulin can be human, humanized, or non-human (*e.g*., a murine framework modified to decrease antigenicity in humans), or a synthetic framework (*e.g*., a consensus sequence). Humanized immunoglobulins are those in which the framework residues correspond to human germline sequences and the CDRs result from V(D)J recombination and somatic mutations. However, humanized immunoglobulins may also comprise amino acid residues not encoded in human germline immunoglobulin nucleic acid sequences (*e.g*., mutations introduced by random or site-specific mutagenesis *ex vivo*)*.* It has been demonstrated that *in vivo* somatic mutation of human variable genes results in mutation of framework residues (see Nature Immunol. 2:537 (2001)). Such an antibody would be termed "human" given its source, despite the framework mutations. Mouse antibody variable domains also contain somatic mutations in framework residues (See Sem. Immunol. 8:159 (1996)). Consequently, transgenic mice containing the human Ig locus produce immunoglobulins that are commonly referred to as "fully human," even though they possess an average of 4.5 framework mutations (Nature Genet. 15:146-56 (1997)). Accepted usage therefore indicates that an antibody variable domain gene based on germline sequence but possessing framework mutations introduced by, for example, an *in vivo* somatic mutational process is termed "human."

Humanized antibodies may be engineered by a variety of methods known in the art including, for example: (1) grafting the non-human complementarity determining regions (CDRs) onto a human framework and constant region (a process referred to in the art as humanizing), or, alternatively, (2) transplanting the entire non-human variable domains, but providing them with a human-like surface by replacement of surface residues (a process referred to in the art as veneering). Humanized antibodies can include both humanized and veneered antibodies. Similarly, human antibodies can be made by introducing human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); Lonberg and Huszar, Intern. Rev. Immunol. 13:65-93 (1995); Jones et al., Nature 321:522-525 (1986); Morrison et al., Proc. Natl. Acad. Sci, USA, 81:6851-6855 (1984); Morrison and Oi, Adv. Immunol., 44:65-92 (1988); Verhoeyer et al., Science 239:1534-1536 (1988); Padlan, Molec. Immun. 28:489-498 (1991); Padlan, Molec. Immunol. 31(3):169-217 (1994); and Kettleborough, C. A. et al., Protein Eng. 4(7):773-83 (1991)).

In addition to chimeric and humanized antibodies, fully human antibodies can be derived from transgenic mice having human immunoglobulin genes (see, *e.g.,* U.S. Patent Nos. 6,075,181; 6,091,001; and 6,114,598), or from phage display libraries of human immunoglobulin genes (see, *e.g.* McCafferty et al., Nature 348:552-554 (1990); Clackson et al., Nature 352:624-628 (1991), and Marks et al., J. Mol. Biol. 222:581-597 (1991)). In some embodiments, antibodies may be produced and identified by scFv-phage display libraries using standard methods known in the art.

The anti-sEcad antibodies may be modified to modulate their antigen binding affinity, their effector functions, or their pharmacokinetics. In particular, random mutations can be made in the CDRs and products screened to identify antibodies with higher affinities and/or higher specificities. Such mutagenesis and selection is routinely practiced in the antibody arts. A convenient way for generating such substitutional variants is affinity maturation using phage display.

CDR shuffling and implantation technologies can be used with the antibodies provided herein, for example. CDR shuffling inserts CDR sequences into a specific framework region (Jirholt et al., Gene 215:471 (1988)). CDR implantation techniques permit random combination of CDR sequences into a single master framework (Soderlind et al., Immunotechnol. 4:279 (1999); and Soderlind et al., Nature Biotechnol. 18:852 (2000)). Using such techniques, CDR sequences of the anti- sEcad antibody, for example, can be mutagenized to create a plurality of different sequences, which can be incorporated into a scaffold sequence and the resultant antibody variants screened for desired characteristics, *e.g*., higher affinity. In some embodiments, sequences of the anti-sEcad antibody can be examined for the presence of T cell epitopes, as is known in the art. The underlying sequence can then be changed to remove T cell epitopes, *i.e.,* to "deimmunize" the antibody.

Recombinant technology using, for example phagemid technology, allows for preparation of antibodies having a desired specificity from recombinant genes encoding a range of antibodies. Certain recombinant techniques involve isolation of antibody genes by immunological screening of combinatorial immunoglobulin phage expression libraries prepared from RNA isolated from spleen of an immunized animal (Morrison et al., Mt. Sinai J. Med. 53:175 (1986); Winter and Milstein, Nature 349:293 (1991); Barbas et al., Proc. Natl. Acad. Sci. USA 89:4457 (1992)). For such methods, combinatorial immunoglobulin phagemid libraries can be prepared from RNA isolated from spleen of an immunized animal, and phagemids expressing appropriate antibodies can be selected by panning using cells expressing antigen and control cells. Advantage of this approach over conventional hybridoma techniques include approximately 10⁴ times as many antibodies can be produced and screened in a single round, and that new specificities can be generated by H and L chain combination, which can further increase the percentage of appropriate antibodies generated.

One method for the generation of a large repertoire of diverse antibody molecules in bacteria utilizes the bacteriophage lambda as the vector (Huse et al., Science 246:1275 (1989)). Production of antibodies using the lambda vector involves the cloning of heavy and light chain populations of DNA sequences into separate starting vectors. Vectors subsequently can be randomly combined to form a single vector that directs co-expression of heavy and light chains to form antibody fragments. The general technique for filamentous phage display is described (U.S. Patent No. 5,658,727). In a most general sense, the method provides a system for the simultaneous cloning and screening of pre-selected ligand-binding specificities from antibody gene repertoires using a single vector system. Screening of isolated members of the library for a pre-selected ligand-binding capacity allows the correlation of the binding capacity of an expressed antibody molecule with a convenient means to isolate a gene that encodes the member from the library. Additional methods for screening phagemid libraries are described (U.S. Patent Nos. 5,580,717; 5,427,908; 5,403,484; and 5,223,409).

One method for the generation and screening of large libraries of wholly or partially synthetic antibody combining sites, or paratopes, utilizes display vectors derived from filamentous phage such as M13, fl or fd (U.S. Patent No. 5,698,426). Filamentous phage display vectors, referred to as "phagemids," yield large libraries of monoclonal antibodies having diverse and novel immunospecificities. The technology uses a filamentous phage coat protein membrane anchor domain as a means for linking gene-product and gene during the assembly stage of filamentous phage replication, and has been used for the cloning and expression of antibodies from combinatorial libraries (Kang et al., Proc. Natl. Acad. Sci. USA 88:4363 (1991); and Barbas et al., Proc. Natl. Acad. Sci. USA 88:7978 (1991)). The surface expression library is screened for specific Fab fragments that bind neuraminidase molecules by standard affinity isolation procedures. The selected Fab fragments can be characterized by sequencing the nucleic acids encoding the polypeptides after amplification of the phage population.

One method for producing diverse libraries of antibodies and screening for desirable binding specificities is described (U.S. Patent Nos. 5,667,988 and 5,759,817). The method involves the preparation of libraries of heterodimeric immunoglobulin molecules in the form of phagemid libraries using degenerate oligonucleotides and primer extension reactions to incorporate degeneracies into CDR regions of immunoglobulin variable heavy and light chain variable domains, and display of mutagenized polypeptides on the surface of the phagemid. Thereafter, the display protein is screened for the ability to bind to a preselected antigen. A further variation of this method for producing diverse libraries of antibodies and screening for desirable binding specificities is described U.S. Patent No. 5,702,892. In this method, only heavy chain sequences are employed, heavy chain sequences are randomized at all nucleotide positions that encode either the CDRI or CDRIII hypervariable region, and the genetic variability in the CDRs can be generated independent of any biological process.

In addition to the combinatorial immunoglobulin phage expression libraries disclosed above, one molecular cloning approach is to prepare antibodies from transgenic mice containing human antibody libraries. Such techniques are described (U.S. Patent No. 5,545,807,). Such transgenic animals can be employed to produce human antibodies of a single isotype, more specifically an isotype that is essential for B cell maturation, such as IgM and possibly IgD. Another method for producing human antibodies is described in U.S. Patent Nos. 5,545,806; 5,569,825; 5,625,126; 5,633,425; 5,661,016; and 5,770,429, wherein transgenic animals are described that are capable of switching from an isotype needed for B cell development to other isotypes.

The anti-sEcad immunoglobulins may be modified to reduce or abolish glycosylation. An immunoglobulin that lacks glycosylation may be an immunoglobulin that is not glycosylated at all; that is not fully glycosylated; or that is atypically glycosylated (*i.e.,* the glycosylation pattern for the mutant differs from the glycosylation pattern of the corresponding wild type immunoglobulin). The IgG polypeptides include one or more (*e.g.,* 1, 2, or 3 or more) mutations that attenuate glycosylation, *i.e.,* mutations that result in an IgG CH2 domain that lacks glycosylation, or is not fully glycosylated or is atypicially glycosylated. Mutations of the asparagine residue at amino acid 297 in human IgG1 is an example of such a mutation. The oligosaccharide structure can also be modified, for example, by eliminating the fusose moiety from the N-linked glycan.

Antibodies can also be modified to increase their stability and or solubility in vivo by conjugation to non-protein polymers, e.g, polyethylene glycol. Any PEGylation method can be used as long as the anti-sEcad antibody retains the ability to selectively bind the second, third, fourth or fifth subdomain of sEcad.

A wide variety of antibody/immunoglobulin frameworks or scaffolds can be employed so long as the resulting polypeptide includes at least one binding region that is specific for the target, *i.e.,* the second, third, fourth, or fifth subdomain of sEcad. Such frameworks or scaffolds include the five main idiotypes of human immunoglobulins, or fragments thereof (such as those disclosed elsewhere herein), and include immunoglobulins of other animal species, preferably having humanized aspects. Single heavy-chain antibodies such as those identified in camelids are of particular interest in this regard.

One can generate non-immunoglobulin based antibodies using non-immunoglobulin scaffolds onto which CDRs of the sEcad antibody can be grafted. Any non-immunoglobulin framework and scaffold know to those in the art may be used, as long as the framework or scaffold includes a binding region specific for the target. Immunoglobulin-like molecules include proteins that share certain structural features with immunoglobulins, for example, a β-sheet secondary structure. Examples of non-immunoglobulin frameworks or scaffolds include, but are not limited to, adnectins (fibronectin), ankyrin, domain antibodies and Ablynx nv, lipocalin, small modular immuno-pharmaceuticals (Trubion Pharmaceuticals Inc., Seattle, WA), maxybodies (Avidia, Inc., Mountain View, CA), Protein A and affilin (gamma-crystallin or ubiquitin) (Scil Proteins GmbH, Halle, Germany).

The anti-sEcad antibodies of the invention specifically bind to an epitope on the second, third, fourth or fifth subdomain of sEcad (but not to an epitope of EC1). An epitope refers to an antigenic determinant on a target that is specifically bound by the paratope, *i.e.,* the binding site of an antibody. Epitopic determinants usually consist of chemically active surface groupings of molecules such as amino acids or sugar side chains, and typically have specific three-dimensional structural characteristics, as well as specific charge characteristics. Epitopes generally have between about 4 to about 10 contiguous amino acids (a continuous epitope), or alternatively can be a set of noncontiguous amino acids that define a particular structure (*e.g*., a conformational epitope). Thus, an epitope can consist of at least 4, at least 6, at least 8, at least 10, and at least 12 such amino acids. Methods of determining the spatial conformation of amino acids are known in the art, and include, for example, x-ray crystallography and 2-dimensional nuclear magnetic resonance.

Methods of predicting other potential epitopes to which an antibody can bind are well-known to those of skill in the art and include without limitation, Kyte-Doolittle Analysis (Kyte and Dolittle, J. Mol. Biol. 157:105-132 (1982)), Hopp and Woods Analysis (Hopp and Woods, Proc. Natl. Acad. Sci. USA 78:3824-3828 (1981); Hopp and Woods, Mol. Immunol. 20:483-489 (1983); Hopp, J. Immunol. Methods 88:1-18 (1986)), Jameson-Wolf Analysis (Jameson and Wolf Comput. Appl. Biosci. 4:181-186 (1988)), and Emini Analysis (Emini et al., Virology 140:13-20 (1985)). Potential epitopes may be identified by determining theoretical extracellular domains. Analysis algorithms such as TMpred *(see* Hofmann and Stoffel, Biol. Chem. 374:166 (1993)) or TMHMM (Krogh et al., J. Mol. Biol., 305(3):567-580 (2001)) can be used to make such predictions. Other algorithms, such as SignalP 3.0 (Bednsten et al., J. Mol. Biol. 340(4):783-795 (2004)) can be used to predict the presence of signal peptides and to predict where those peptides would be cleaved from the full-length protein. The portions of the proteins on the outside of the cell can serve as targets for antibody interaction.

The compositions of the present invention include antibodies that (1) exhibit a threshold level of binding activity; and/or (2) do not significantly cross-react with known related polypeptide molecules. The binding affinity of an antibody can be readily determined by one of ordinary skill in the art, for example, by Scatchard analysis (Scatchard, Ann. NY Acad, Sci. 51:660-672 (1949)).

In some embodiments, the anti-sEcad antibodies can bind to their target epitopes or mimetic decoys at least 1.5-fold, 2-fold, 5-fold 10-fold, 100-fold, 10³-fold, 10⁴-fold, 10⁵-fold, 10⁶-fold or greater for the target second, third, fourth or fifth subdomain of sEcad than to other proteins predicted to have some homology to the second, third, fourth or fifth subdomain of sEcad.

In some embodiments the anti-sEcad antibodies bind with high affinity of 10⁻⁴M or less, 10⁻⁷M or less, 10⁻⁹M or less or with subnanomolar affinity (0.9, 0.8, 0.7, 0.6, 0.5, 0.4, 0.3, 0.2, 0.1 nM or even less). In some embodiments the binding affinity of the antibodies for the second, third, fourth or fifth subdomain of sEcad is at least 1 x 10⁶ Ka. In some embodiments the binding affinity of the antibodies for the second, third, fourth or fifth subdomain of sEcad is at least 5x10⁶ Ka, at least 1x10⁷ Ka, at least 2x10⁷ Ka, at least 1x10⁸ Ka, or greater. Antibodies may also be described or specified in terms of their binding affinity to the second, third, fourth or fifth subdomain of sEcad. In some embodiments binding affinities include those with a Kd less than 5x10⁻² M, 10⁻² M, 5x10⁻³ M, 10⁻³ M, 5x10⁻³ M, 10⁻⁴ M, 5x10⁻⁵ M, 10⁻⁵ M, 5x10.⁻⁶ M, 10⁻⁶ M, 5x10⁻⁷ M, 10.⁻⁷ M, 5x10.⁻⁸ M, 10⁻⁸ M, 5x10.⁻⁹ M, 5x10.⁻¹⁰ M, 10⁻¹⁰ M, 5x10⁻¹¹ M, 10⁻¹¹M, 5x10⁻¹²M, 10⁻¹² M, 5x10⁻¹³ M, 10⁻¹³ M, 5x10⁻¹⁴ M, 10⁻¹⁴ M, 5x10⁻¹⁵ M, or 10⁻¹⁵ M, or less.

In some embodiments, the antibodies do not bind to known related polypeptide molecules; for example, they bind the second, third, fourth or fifth subdomain of a sEcad polypeptide but not known related polypeptides. Antibodies may be screened against known related polypeptides to isolate an antibody population that specifically binds to second, third, fourth or fifth subdomain of a sEcad polypeptide. For example, antibodies specific to second, third, fourth or fifth subdomain of a sEcad polypeptide will flow through a column comprising second, third, fourth or fifth subdomain of a sEcad polypeptide -related proteins (with the exception of second, third, fourth or fifth subdomain of a sEcad polypeptide) adhered to insoluble matrix under appropriate buffer conditions. Such screening allows isolation of polyclonal and monoclonal antibodies non-crossreactive to closely related polypeptides (Antibodies: A Laboratory Manual, Harlow and Lane (eds.), Cold Spring Harbor Laboratory Press, 1988; Current so Protocols in Immunology, Cooligan et al. (eds.), National Institutes of Health, John Wiley and Sons, Inc., 1995). Screening and isolation of specific antibodies is well known in the art (see, Fundamental Immunology, Paul (eds.), Raven Press, 1993; Getzoff et al., Adv. in Immunol. 43:1-98 (1988); Monoclonal Antibodies: Principles and Practice, Goding, J. W. (eds.), Academic Press Ltd., 1996; Benjamin et al., Ann. Rev. Immunol. 2:67-101, 1984). Representative examples of such assays include: concurrent immunoelectrophoresis, radioimmunoassay (RIA), radioimmunoprecipitation, enzyme-linked immunosorbent assay (ELISA), dot blot or Western blot assay, inhibition or competition assay, and sandwich assay.

The ability of a particular antibody to selectively kill malignant e-cadherin expressing cells can be evaluated using, for example, the methods disclosed in the Examples herein.

The anti-sEcad antibodies can include a tag, which may also be referred to as a reporter or marker (*e.g.,* a detectable marker). A detectable marker can be any molecule that is covalently linked to anti-sEcad antibody or a biologically active fragment thereof that allows for qualitative and/or quantitative assessment of the expression or activity of the tagged peptide. The activity can include a biological activity, a physico-chemical activity, or a combination thereof. Both the form and position of the detectable marker can vary, as long as the labeled antibody retains biological activity. Many different markers can be used, and the choice of a particular marker will depend upon the desired application. Labeled anti-sEcad antibodies can be used, for example, for assessing the levels of sEcad in a biological sample, *e.g*., urine, salive, cerebrospinal fluid, blood or a biopsy sample or for evaluation the clinical response to sEcad peptide therapeutics.

Suitable markers include, for example, enzymes, photo-affinity ligands, radioisotopes, and fluorescent or chemiluminescent compounds. Methods of introducing detectable markers into peptides are well known in the art. Markers can be added during synthesis or post-synthetically. Recombinant anti-sEcad antibodies or biologically active variants thereof can also be labeled by the addition of labeled precursors (*e.g*., radiolabeled amino acids) to the culture medium in which the transformed cells are grown. In some embodiments, analogues or variants of peptides can be used in order to facilitate incorporation of detectable markers. For example, any N-terminal phenylalanine residue can be replaced with a closely related aromatic amino acid, such as tyrosine, that can be easily labeled with ¹²⁵I. In some embodiments, additional functional groups that support effective labeling can be added to the fragments of an anti-sEcad antibody or biologically active variants thereof. For example, a 3-tributyltinbenzoyl group can be added to the N-terminus of the native structure; subsequent displacement of the tributyltin group with ¹²⁵I will generate a radiolabeled iodobenzoyl group.

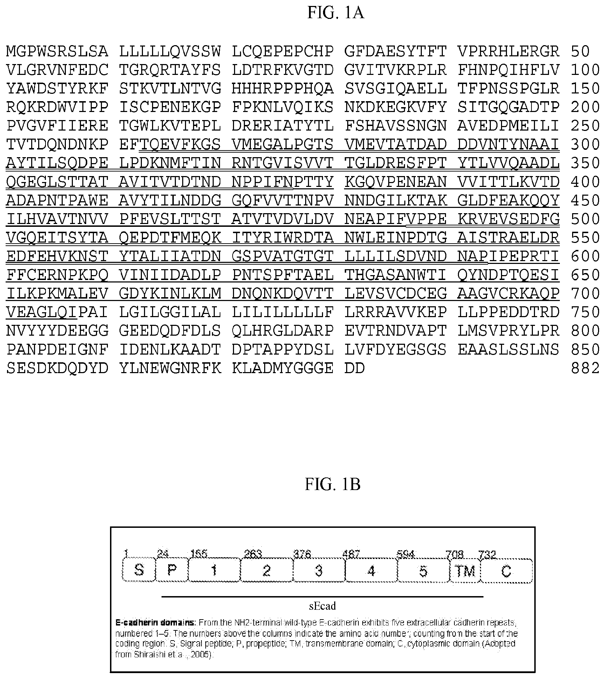

The methods may also be carried out by administering a protein that elicits the production of anti-sEcad antibodies *in vivo.* Accordingly, the compositions described include antigenic fragments of the extracellular domain of E-cadherin in the EC2-EC5 subdomains (*see* FIG 1A and FIG. 1B). These polypeptides can be fused to a heterologous polypeptide to generate an immunogenic fusion protein. For example, an sEcad polypeptide can be fused to a fragment of the influenza virus HA2 hemagglutinin protein as described in U.S. Patent No. 7,262,270.

Also described are nucleic acids that can be used to inhibit the expression of E-cadherin (*e.g.,* an antisense oligonucleotide or an oligonucleotide that mediates RNA interference). Nucleic acid constructs can also be used to express antigenic fragments of sEcad *in vivo* or *ex vivo* (*e.g.,* in cell or tissue culture).

The terms "nucleic acid" and "polynucleotide" may be used interchangeably herein, and refer to both RNA and DNA, including cDNA, genomic DNA, synthetic DNA, and DNA (or RNA) containing nucleic acid analogs. Polynucleotides can have any three-dimensional structure. A nucleic acid can be double-stranded or single-stranded (*i.e.,* a sense strand or an antisense strand). Non-limiting examples of polynucleotides include genes, gene fragments, exons, introns, messenger RNA (mRNA) and portions thereof, transfer RNA, ribosomal RNA, siRNA, micro-RNA, ribozymes, cDNA, recombinant polynucleotides, branched polynucleotides, plasmids, vectors, isolated DNA of any sequence, isolated RNA of any sequence, nucleic acid probes, and primers, as well as nucleic acid analogs. In the context of the present invention, nucleic acids can encode, for example, an antibody, a mutant antibody or fragment thereof or a sEcad or fragment thereof.

An "isolated" nucleic acid can be, for example, a naturally-occurring DNA molecule or a fragment thereof, provided that at least one of the nucleic acid sequences normally found immediately flanking that DNA molecule in a naturally-occurring genome is removed or absent. Thus, an isolated nucleic acid includes, without limitation, a DNA molecule that exists as a separate molecule, independent of other sequences (*e.g*., a chemically synthesized nucleic acid, or a cDNA or genomic DNA fragment produced by the polymerase chain reaction (PCR) or restriction endonuclease treatment). An isolated nucleic acid also refers to a DNA molecule that is incorporated into a vector, an autonomously replicating plasmid, a virus, or into the genomic DNA of a prokaryote or eukaryote. In addition, an isolated nucleic acid can include an engineered nucleic acid such as a DNA molecule that is part of a hybrid or fusion nucleic acid. A nucleic acid existing among many (*e.g.*, dozens, or hundreds to millions) of other nucleic acids within, for example, cDNA libraries or genomic libraries, or gel slices containing a genomic DNA restriction digest, is not an isolated nucleic acid.

Isolated nucleic acid molecules can be produced by standard techniques. For example, polymerase chain reaction (PCR) techniques can be used to obtain an isolated nucleic acid containing a nucleotide sequence described herein, including nucleotide sequences encoding a polypeptide described herein (*i.e.* an engineered protein). PCR can be used to amplify specific sequences from DNA as well as RNA, including sequences from total genomic DNA or total cellular RNA. Various PCR methods are described in, for example, PCR Primer: A Laboratory Manual, Dieffenbach and Dveksler, eds., Cold Spring Harbor Laboratory Press, 1995. Generally, sequence information from the ends of the region of interest or beyond is employed to design oligonucleotide primers that are identical or similar in sequence to opposite strands of the template to be amplified. Various PCR strategies also are available by which site-specific nucleotide sequence modifications can be introduced into a template nucleic acid (as one may wish to do, for example, when making an engineered protein, for example, an antibody, a mutant antibody or fragment thereof, or a fusion protein or fragment thereof. Isolated nucleic acids also can be chemically synthesized, either as a single nucleic acid molecule (*e.g*., using automated DNA synthesis in the 3' to 5' direction using phosphoramidite technology) or as a series of oligonucleotides. For example, one or more pairs of long oligonucleotides (*e.g.,* >50-100 nucleotides) can be synthesized that contain the desired sequence, with each pair containing a short segment of complementarity (*e.g*., about 15 nucleotides) such that a duplex is formed when the oligonucleotide pair is annealed. DNA polymerase is used to extend the oligonucleotides, resulting in a single, double-stranded nucleic acid molecule per oligonucleotide pair, which then can be ligated into a vector. Isolated nucleic acids also can be obtained by mutagenesis of, for example, a naturally occurring portion of an engineered protein-encoding DNA.

The nucleic acids and polypeptides described (*e.g*., antigenic fragments of sEcad) may be referred to as "exogenous". The term "exogenous" indicates that the nucleic acid or polypeptide is part of, or encoded by, a recombinant nucleic acid construct, or is not in its natural environment. For example, an exogenous nucleic acid can be a sequence from one species introduced into another species, *i.e.,* a heterologous nucleic acid. Typically, such an exogenous nucleic acid is introduced into the other species *via* a recombinant nucleic acid construct. An exogenous nucleic acid can also be a sequence that is native to an organism and that has been reintroduced into cells of that organism. An exogenous nucleic acid that includes a native sequence can often be distinguished from the naturally occurring sequence by the presence of non-natural sequences linked to the exogenous nucleic acid, *e.g*., non-native regulatory sequences flanking a native sequence in a recombinant nucleic acid construct. In addition, stably transformed exogenous nucleic acids typically are integrated at positions other than the position where the native sequence is found.

Recombinant constructs are also described and can be used to transform cells in order to express a polypeptide, for example, an antibody, a mutant antibody or fragment thereof, or a sEcad or fragment thereof. A recombinant nucleic acid construct comprises a nucleic acid encoding, for example, an antibody, a mutant antibody or fragment thereof or a sEcad or fragment thereof as described herein, operably linked to a regulatory region suitable for expressing the engineered protein, for example, an antibody, a mutant antibody or fragment thereof or a sEcad or fragment thereof. In some cases, a recombinant nucleic acid construct can include a nucleic acid comprising a coding sequence, a gene, or a fragment of a coding sequence or gene in an antisense orientation so that the antisense strand of RNA is transcribed. It will be appreciated that a number of nucleic acids can encode a polypeptide having a particular amino acid sequence. The degeneracy of the genetic code is well known in the art. For many amino acids, there is more than one nucleotide triplet that serves as the codon for the amino acid. For example, codons in the coding sequence for a given fragment of an antibody, a mutant antibody or fragment thereof, or a fusion protein or fragment thereof can be modified such that optimal expression in a particular organism is obtained, using appropriate codon bias tables for that organism.

Vectors containing nucleic acids are described. A "vector" is a replicon, such as a plasmid, phage, or cosmid, into which another DNA segment may be inserted so as to bring about the replication of the inserted segment. Expression vectors include plasmid vectors, viral vectors, and the HSV amplicon particles as described in U.S. Application Publication No. 2006/0239970

Generally, a vector is capable of replication when associated with the proper control elements. Suitable vector backbones include, for example, those routinely used in the art such as plasmids, viruses, artificial chromosomes, BACs, YACs, or PACs. The term "vector" includes cloning and expression vectors, as well as viral vectors and integrating vectors. An "expression vector" is a vector that includes a regulatory region. Suitable expression vectors include, without limitation, plasmids and viral vectors derived from, for example, bacteriophage, baculoviruses, and retroviruses. Numerous vectors and expression systems are commercially available from such corporations as Novagen (Madison, WI), Clontech (Palo Alto, CA), Stratagene (La Jolla, CA), and Invitrogen/Life Technologies (Carlsbad, CA).

The vectors also can include, for example, origins of replication, scaffold attachment regions (SARs), and/or markers. A marker gene can confer a selectable phenotype on a host cell. For example, a marker can confer biocide resistance, such as resistance to an antibiotic (*e.g*., kanamycin, G418, bleomycin, or hygromycin). As noted above, an expression vector can include a tag sequence designed to facilitate manipulation or detection (*e.g*., purification or localization) of the expressed polypeptide. Tag sequences, such as green fluorescent protein (GFP), glutathione S-transferase (GST), polyhistidine, c-myc, hemagglutinin, or Flag™ tag (Kodak, New Haven, CT) sequences typically are expressed as a fusion with the encoded polypeptide. Such tags can be inserted anywhere within the polypeptide, including at either the carboxyl or amino terminus.

The vector can also include a regulatory region. The term "regulatory region" refers to nucleotide sequences that influence transcription or translation initiation and rate, and stability and/or mobility of a transcription or translation product. Regulatory regions include, without limitation, promoter sequences, enhancer sequences, response elements, protein recognition sites, inducible elements, protein binding sequences, 5' and 3' untranslated regions (UTRs), transcriptional start sites, termination sequences, polyadenylation sequences, and introns.

As used herein, the term "operably linked" refers to positioning of a regulatory region and a sequence to be transcribed in a nucleic acid so as to influence transcription or translation of such a sequence. For example, to bring a coding sequence under the control of a promoter, the translation initiation site of the translational reading frame of the polypeptide is typically positioned between one and about fifty nucleotides downstream of the promoter. A promoter can, however, be positioned as much as about 5,000 nucleotides upstream of the translation initiation site or about 2,000 nucleotides upstream of the transcription start site. A promoter typically comprises at least a core (basal) promoter. A promoter also may include at least one control element, such as an enhancer sequence, an upstream element or an upstream activation region (UAR). The choice of promoters to be included depends upon several factors, including, but not limited to, efficiency, selectability, inducibility, desired expression level, and cell- or tissue-preferential expression. It is a routine matter for one of skill in the art to modulate the expression of a coding sequence by appropriately selecting and positioning promoters and other regulatory regions relative to the coding sequence.

Also described are host cells. A host cell can be for example, a prokaryote *e.g*., a bacterium such as *E. coli,* or a eukaryote, *e.g.,* yeast, insect or mammalian cell that expresses a polypeptide described.

The agents described herein that inhibit sEcad can be included in pharmaceutical compositions that are physiologically acceptable (*i.e.,* sufficiently non-toxic to be used in the therapeutic and prophylactic methods described herein). Accordingly, the invention features a variety of formulations, including topical creams (integrated into sunscreens) and sustained-release patches for transdermal delivery sEcad inhibitors. In other embodiments, the pharmaceutical composition can be formulated as an oral rinse, gel, or emulsion, or as a rectal solution, suspension, or emulsion. As will be apparent to one of ordinary skill in the art, the specific formulations can be selected based on the type of cancer being treated. For example, the oral rinse, gel, or emulsion can be used to treat cancers in the mouth, throat, esophagus, or stomach, and the rectal solution, suspension, or emulsion can be used to treat cancers in the rectum or colon.

The therapeutic antibodies of the invention can be formulated for administration to a patient with materials that improve their stability and/or provide for a controlled or sustained release *in vivo.* Accordingly, the invention encompasses delivery systems in which an sEcad-specific agent is formulated with microparticles (*e.g*., polymeric microparticles such as polylactide-co-glycolide microparticles) or nanoparticles (*e.g*., liposomes, polymeric carbohydrate nanoparticles, dendrimers, and carbon-based nanoparticles).

Other formulations include those for subcutaneous, intraperitoneal, intravenous, intraarterial, or pulmonary administration. As noted, sustained-release implants can also be made and used.

Any of the therapeutic or prophylactic methods described can include a step of assessing a patient prior to treatment or as treatment progresses. As noted above, it is well documented that sEcad is elevated in the urine and/or serum of patients with breast, skin, lung, prostate, gastric and colorectal cancers as well as other epithelial malignancies. Consistent with earlier studies on sEcad levels, our data demonstrate that sEcad is shed at low levels from the surface of normal epithelial cells and at much higher levels from human skin cancer cells, human breast cancer cells, and mouse lung cancer cells. Thus, the methods can include a step in which sEcad levels are determined from a sample (*e.g.,* a urine or blood sample) obtained from a subject. Elevated levels are an indication that a subject is a good candidate for treatment as described herein, and monitoring sEcad as treatment progresses can help optimize dosing and scheduling as well as predict outcome. For example, monitoring can be used to detect the onset of resistance and to rapidly distinguish responsive patients from nonresponsive patients. Where there are signs of resistance or nonresponsivness, a physician can choose an alternative or adjunctive agent before the tumor develops additional escape mechanisms.

Compositions comprising two or more agents that specifically target one or more of the second, third, fourth or fifth subdomains of sEcad may be administered to persons or mammals suffering from, or predisposed to suffer from, cancer. The anti-sEcad antibodies may also be administered with another therapeutic agent, such as a cytotoxic agent, or cancer chemotherapeutic. Concurrent administration of two or more therapeutic agents does not require that the agents be administered at the same time or by the same route, as long as there is an overlap in the time period during which the agents are exerting their therapeutic effect. Simultaneous or sequential administration is contemplated, as is administration on different days or weeks.

The pharmaceutical compositions of the present invention can also include, in addition to an sEcad targeting antibody, another therapeutic antibody (or antibodies (*e.g*., antibodies that recognize a cellular target (or targets) other than sEcad)). Exemplary immunoglobulins are listed below. Each immunoglobulin is identified by its proper name and its trade name. Numbers in parenthesis beginning with "DB" refer to the identifiers for each antibody on The DrugBank database available at the University of Alberta. The DrugBank database is described in Wishart et al., Nucl. Acids Res. 36:D901-906 (2008)) on the world wide web at www.drugbank.ca. Useful immunoglobulins include: Abciximab (ReoPro™) (DB00054), the Fab fragment of the chimeric human-murine monoclonal antibody 7E3, the synthesis of which is described in EP0418316 (A1) and WO 89/11538 (A1); Adalimumab (Humira™) (DB00051), a fully human monoclonal antibody that binds to Tumor Necrosis Factor alpha (TNF-alpha) and blocks TNF-alpha binding to its cognate receptor; alemtuzumab (Campath™) (DB00087), a humanized monoclonal antibody that targets CD52, a protein present on the surface of mature lymphocytes, used in the treatment of chronic lymphocytic leukemia (CLL), cutaneous T cell lymphoma (CTCL) and T-cell lymphoma; basiliximab (Simulect™) (DB00074), a chimeric mouse-human monoclonal antibody to the alpha chain (CD25) of the IL-2 receptor; bevacizumab (Avastin™) (DB00112) a humanized monoclonal antibody that recognises and blocks vascular endothelial growth factor (VEGF), the chemical signal that stimulates angiogenesis, the synthesis of which is described in Presta et al., Cancer Res., 57:4593-4599 (1997); certuximab (Erbitux™) (DB00002), a chimeric (mouse/human) monoclonal antibody that binds to and inhibits the epidermal growth factor receptor (EGFR), the synthesis of which is described in U.S. Patent No. 6,217,866; certolizumab pegol (Cimzia™), a PEGylated Fab' fragment of a humanized TNF inhibitor monoclonal antibody; daclizumab (Zenapax™) (DB00111), a humanized monoclonal antibody to the alpha subunit of the IL-2 receptor; eculizumab (Soliris™), a humanized monoclonal antibody that binds to the human C5 complement protein; efalizumab (Raptiva™) (DB00095), a humanized monoclonal antibody that binds to CD11a; gemtuzumab (Mylotarg™) (DB00056) a monoclonal antibody to CD33 linked to a cytotoxic agent, the amino acid sequence of which is described in J. Immunol. 148:1149 (1991), and Caron et al., Cancer. 73(3 Suppl): 1049-1056 (1994)); ibritumomab tiuxetan (Zevalin™) (DB00078), a monoclonal mouse IgG1 antibody ibritumomab in conjunction with the chelator tiuxetan and a radioactive isotope (yttrium⁹⁰ or indium¹¹¹); Infliximab (Remicade™) (DB00065), a chimeric mouse-human monoclonal antibody that binds to tumour necrosis factor alpha (TNF-alpha), the synthesis of which is described in U.S. Patent No. 6,015,557; muromonab-CD3 (Orthoclone OKT3™), a mouse monoclonal IgG2a antibody that binds to the T cell receptor-CD3-complex; natalizumab (Tysabri™) (DB00108), a humanized monoclonal antibody against the cellular adhesion molecule α4-integrin, the sequence of which is described in Leger et al., Hum. Antibodies 8(1):3-16 (1997); omalizumab (Xolair™) (DB00043), a humanized IgGlk monoclonal antibody that selectively binds to human immunoglobulin E (IgE); palivizumab (Synagis™) (DB00110), a humanized monoclonal antibody (IgG) directed against an epitope in the A antigenic site of the F protein of the Respiratory Syncytial Virus (RSV), the amino acid sequence of which is described in Johnson et al., J. Infect. Dis. 176(5):1215-1224 (1997); panitumumab (Vectibix™), a fully human monoclonal antibody specific to the epidermal growth factor receptor (also known as EGF receptor, EGFR, ErbB-1 and HER1 in humans); ranibizumab (Lucentis™), an affinity matured anti-VEGF-A monoclonal antibody fragment derived from the same parent murine antibody as bevacizumab (Avastin™); rituximab (Rituxan™, Mabthera™) (DB00073), a chimeric monoclonal antibody against the protein CD20, which is primarily found on the surface of B cells; tositumomab (Bexxar™) (DB00081), an anti-CD20 mouse monoclonal antibody covalently bound to ¹³¹I; or trastuzumab (Herceptin™) (DB00072), a humanized monoclonal antibody that binds selectively to the HER2 protein.

The antibodies can include bioequivalents of the approved or marketed antibodies (biosimilars). A biosimilar can be for example, a presently known antibody having the same primary amino acid sequence as a marketed antibody, but one that may be made in a different cell type or by a different production, purification or formulation method than the marketed antibody. Generally, any deposited materials can be used.

The pharmaceutical compositions may also include or be administered along with a cytotoxic agent, *e.g.,* a substance that inhibits or prevents the function of cells and/or causes destruction of cells. Exemplary cytotoxic agents include radioactive isotopes (*e.g*., ¹³¹I, ¹²⁵I, ⁹⁰Y and ¹⁸⁶Re), chemotherapeutic agents, and toxins such as enzymatically active toxins of bacterial, fungal, plant or animal origin or synthetic toxins, or fragments thereof. A non-cytotoxic agent refers to a substance that does not inhibit or prevent the function of cells and/or does not cause destruction of cells. A non-cytotoxic agent may include an agent that can be activated to be cytotoxic. A non-cytotoxic agent may include a bead, liposome, matrix or particle (see, *e.g.,* U.S. Patent Publications 2003/0028071 and 2003/0032995). Such agents may be conjugated, coupled, linked or associated with an antibody disclosed herein.

Conventional cancer medicaments can be administered with the compositions disclosed herein. Useful medicaments include anti-angiogenic agents, *i.e.,* agents block the ability of tumors to stimulate new blood vessel growth necessary for their survival. Any anti-angiogenic agent known to those in the art can be used, including agents such as Bevacizumab (Avastin®, Genentech, Inc.) that block the function of vascular endothelial growth factor (VEGF). Other examples include, without limitation, Dalteparin (Fragmin®), Suramin ABT-510, Combretastatin A4 Phosphate, Lenalidomide, LY317615(Enzastaurin), Soy Isoflavone (Genistein; Soy Protein Isolate) AMG-706, Anti-VEGF antibody, AZD2171, Bay 43-9006 (Sorafenib tosylate), PI-88, PTK787/ZK 222584 (Vatalanib), SU11248 (Sunitinib malate), VEGF-Trap, XL184, ZD6474, Thalidomide, ATN-161, EMD 121974 (Cilenigtide) and Celecoxib (Celebrex®).

Other useful therapeutics include those agents that promote DNA-damage, *e.g*., double stranded breaks in cellular DNA, in cancer cells. Any form of DNA-damaging agent know to those of skill in the art can be used. DNA damage can typically be produced by radiation therapy and/or chemotherapy. Examples of radiation therapy include, without limitation, external radiation therapy and internal radiation therapy (also called brachytherapy). Energy sources for external radiation therapy include x-rays, gamma rays and particle beams; energy sources used in internal radiation include radioactive iodine (iodine¹²⁵ or iodine¹³¹), and from strontium⁸⁹, or radioisotopes of phosphorous, palladium, cesium, iridium, phosphate, or cobalt. Methods of administering radiation therapy are well known to those of ordinary skill in the art.

Examples of DNA-damaging chemotherapeutic agents include, without limitation, Busulfan (Myleran), Carboplatin (Paraplatin), Carmustine (BCNU), Chlorambucil (Leukeran), Cisplatin (Platinol), Cyclophosphamide (Cytoxan, Neosar), Dacarbazine (DTIC-Dome), Ifosfamide (Ifex), Lomustine (CCNU), Mechlorethamine (nitrogen mustard, Mustargen), Melphalan (Alkeran), and Procarbazine (Matulane).

Other standard cancer chemotherapeutic agents include, without limitation, alkylating agents, such as carboplatin and cisplatin; nitrogen mustard alkylating agents; nitrosourea alkylating agents, such as carmustine (BCNU); antimetabolites, such as methotrexate; folinic acid; purine analog antimetabolites, mercaptopurine; pyrimidine analog antimetabolites, such as fluorouracil (5-FU) and gemcitabine (Gemzar®); hormonal antineoplastics, such as goserelin, leuprolide, and tamoxifen; natural antineoplastics, such as aldesleukin, interleukin-2, docetaxel, etoposide (VP-16), interferon alfa, paclitaxel (Taxol®), and tretinoin (ATRA); antibiotic natural antineoplastics, such as bleomycin, dactinomycin, daunorubicin, doxorubicin, daunomycin and mitomycins including mitomycin C; and vinca alkaloid natural antineoplastics, such as vinblastine, vincristine, vindesine; hydroxyurea; aceglatone, adriamycin, ifosfamide, enocitabine, epitiostanol, aclarubicin, ancitabine, nimustine, procarbazine hydrochloride, carboquone, carboplatin, carmofur, chromomycin A3, antitumor polysaccharides, antitumor platelet factors, cyclophosphamide (Cytoxin®), Schizophyllan, cytarabine (cytosine arabinoside), dacarbazine, thioinosine, thiotepa, tegafur, dolastatins, dolastatin analogs such as auristatin, CPT-11 (irinotecan), mitozantrone, vinorelbine, teniposide, aminopterin, carminomycin, esperamicins (See, *e.g.,* U.S. Patent No. 4,675,187), neocarzinostatin, OK-432, bleomycin, furtulon, broxuridine, busulfan, honvan, peplomycin, bestatin (Ubenimex®), interferon-β, mepitiostane, mitobronitol, melphalan, laminin peptides, lentinan, Coriolus versicolor extract, tegafur/uracil, estramustine (estrogen/mechlorethamine).

Additional agents which may be used as therapy for cancer patients include EPO, G-CSF, ganciclovir; antibiotics, leuprolide; meperidine; zidovudine (AZT); interleukins 1 through 18, including mutants and analogues; interferons or cytokines, such as interferons α, β, and γ hormones, such as luteinizing hormone releasing hormone (LHRH) and analogues and, gonadotropin releasing hormone (GnRH); growth factors, such as transforming growth factor-β (TGF-β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor-α & β (TNF-α & β); invasion inhibiting factor-2 (IIF-2); bone morphogenetic proteins 1-7 (BMP 1-7); somatostatin; thymosin-α-1; γ-globulin; superoxide dismutase (SOD); complement factors; and anti-angiogenesis factors.

Useful therapeutic agents include, produgs, *e.g*., precursors or derivative forms of a pharmaceutically active substance that is less cytotoxic or non-cytotoxic to tumor cells compared to the parent drug and is capable of being enzymatically activated or converted into an active or the more active parent form. See, *e.g.,* Wilman, Biochemical Society Transactions, 14:375-382 (1986) and Stella et al., "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, b-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use herein include, but are not limited to, those chemotherapeutic agents described above.

Any method known to those in the art can be used to determine if a particular response is induced. Clinical methods that assess the degree of a particular disease state can be used to determine if a response is induced. The particular methods used to eveluate a response will depend on the nature of the patient's disorder, the patient's age and sex, other drugs being administered and the judgment of the attending clinician.

### EXAMPLES

### Example 1: Sequestration of sEcad induces apoptosis in malignant breast epithelial cells in vitro but not in normal human breast epithelial cells.

E-cadherin ectodomain shedding was first described by Wheelock et al. (J. Cell Biochem. 34:187-202 (1987)), who detected a soluble 80 kDa fragment in the media of MCF-7 breast cancer cells. Since then, cleavage of this sEcad fragment has been described in several different malignant cell types *in vitro* including malignant breast, skin, ovarian, prostate, esophageal, colon, lung and brain cells (Davies et al., Clin. Cancer Res. 7(10):3289-3297 (2001); Gil et al., Gynecol. Oncol. 108(2):361-369 (2008); Ito et al., Oncogene 18(50):7080-7090 (1999); Kantak and Kramer, 1998; Noe et al., J. Cell Sci. 114:111-118 (2001); Ryniers et al., Biol. Chem. 383:159-165 (2002); Symowicz et al., Cancer Res. 67(5):2030-2039 (2007)).

To determine if there are differences in the localization and constitutive shedding of E-cadherin between benign and malignant cells, we stained normal MCF-10A breast epithelial cells and MCF-7 breast cancer cells for E-cadherin visualization by immunoflourescent microscopy. Both cell types were grown on 4-well chamber slides (Nunc) in DMEM supplemented with 10% FBS until confluent. Cells were fixed in 100% methanol, immunostained for E-cadherin and examined by immunofluorescence microscopy (x 20 magnification). Histone H1(red) (Santa Cruz) was used to label nuclei. We also analyzed the supernantants for the shed E-cadherin ectodomain by ELISA. Conditioned media from confluent MCF-10A and MCF-7 cells were collected and analyzed for sEcad levels by ELISA (R&D Systems), as per manufacturer's instructions.

Following exposure to a monoclonal antibody specific for the human E-cadherin subdomain EC1 (SHE78-7, Zymed), we observed E-cadherin immunostaining at sites of cell-cell contact, most notably along the intercellular borders within clusters of cells in both cell lines. In contrast, while the shed soluble E-cadherin ectodomain was detected in the conditioned media of both normal MCF-10A cells and MCF-7 cancer cells, it was markedly increased in the MCF-7 cancer cell line. sEcad levels were significantly different for MCF-10A vs. MCF-7 cells.

Next, confluent MCF-10A and MCF-7 cells were cultured in the presence or absence of an antibody directed against the ectodomain of E-cadherin (DECMA; 20 µg/mL) or pre-immune serum (IgG; 20 µg/mL) for 48 hours and analyzed for apoptosis using the terminal deoxynucleotidyl transferase biotin-dUTP nick end labeling (TUNEL) kit (Promega) according to the manufacturer's instructions. There was no appreciable difference in apoptosis between normal MCF-10A cells that were exposed to the anti-sEcad antibody and untreated or isotype control cells. In contrast, anti-sEcad treated MCF-7cancer cells exhibited a marked increase in cellular apoptosis that was not apparent in untreated cells. To further confirm these findings, we used an apoptosis-specific ELISA assay that measures cytoplasmic histone-associated DNA fragments (mononucleosomes and oligonucleosomes). Cell lysates from MCF-10A and MCF-7 cells cultured in the presence or absence of 20 µg/mL of anti-sEcad Ab or IgG for 48 hours, were analyzed for apoptosis using an apoptosis-specific ELISA for cytoplasmic histone-associated DNA fragments (Cell Death Detection ELISA^{PLUS}, Boehringer Mannheim, Mannheim, Germany) according to the manufacturer's instructions. Results revealed that anti-sEcad treatment dramatically induced apoptosis in MCF-7 cells but had no appreciable effect in MCF-10A cells. Levels of necrosis were unaffected by anti-sEcad treatment in either cell lines. Collectively, these results show that inhibiting or sequestering the shed E-cadherin ectodomain from the cellular milieu selectively induces apoptosis in breast cancer cells while sparing normal healthy breast epithelial cells.

Since activation of p53 leads to the transcriptional regulation of genes important for suppressing tumorigenesis and its specific action is exerted mainly through the triggering of apoptosis (Fuster et al., Trends Mol. Med. 13(5):192-199 (2007)), we next wanted to determine whether p53 plays a role in this anti-sEcad-induced apoptosis. Using western blotting and an antibody directed against p53, we examined the effect of anti-sEcad on the expression of this pro-apoptotic protein in MCF-7 cells. Briefly, 50 µg of lysates were electrophoresed on 4-15% gradient gels (BioRad), transferred to polyvinylidene difluoride (PVDF) membranes, and immunoblotted with anti-p53 (Santa Cruz; 1:400) followed by peroxidase-conjugated secondary antibody (1:3,000) and enhanced chemiluminescence (ECL) detection. Equal loading of protein was verified by G3PDH staining. Upon 20 µg/mL of anti-sEcad treatment, p53 started increasing from 24 hours and reached a maximum level by 48 hours. In contrast, untreated cells or cells treated with the isotype control were devoid of p53 expression. Since the presence of p53 in a tumor correlates with a favorable response to chemotherapy, and low levels of p53 confer drug resistance in cancer cells, we believe that inhibiting sEcad in the tumor milieu may enhance cytotoxicity and lower the resistance that develops with current chemotherapeutic strategies.

### Example 2: Human skin squamous cell carcinomas cells undergo apoptosis after anti-sEcad antibody treatment while normal adult human keratinocytes are spared.

To directly determine if the constitutive shedding of sEcad differs in normal epithelial keratinocyte cultures versus human squamous cell carcinomas (SCCs), we evaluated sEcad levels in normal primary human skin keratinocytes (PHK) and in two different human SCC cell lines (SCC12b and SCC13). PHK, SCC12b and SCC13 cells were grown to ∼80% confluence, and sEcad levels in the culture supernantants were examined by western immunoblotting. Briefly, 60 µL of conditioned media from each cell line were electrophoresed on 4-15% gradient gels (BioRad), transferred to polyvinylidene difluoride (PVDF) membranes, and immunoblotted with anti-E-cadherin (Santa Cruz; 1:600) followed by peroxidase-conjugated secondary antibody (1:3,000) and enhanced chemiluminescence (ECL) detection. Conditioned media from PHK, SCC12b and SCC13 cells were also collected and analyzed for levels of sEcad by ELISA, according to the manufacturer's instructions. We observed an increased expression of sEcad in both SCC cell lines by western blotting. This corresponded to a greater than 3-fold increase in the levels of sEcad in the SCC12b and SCC13 cell lines over the non-cancerous PHK cells by ELISA.

To determine whether anti-sEcad antibodies could induce apoptosis in epithelial cancer cells other than the MCF-7 breast cancer cells described above, we cultured PHK (D033), SCC12b and SCC13 cells to confluency in the presence or absence of anti-sEcad (20 µg/mL; DECMA; Sigma) or pre-immune serum (IgG; "isotype control") for 48 hours and analyzed the cells for apoptosis using the TUNEL assay according to the manufacturer's instructions.

SCC12b and SCC13 cells exhibited a marked increase in apoptosis when exposed to anti-sEcad, whereas treatment of normal PHK with this blocking antibody exhibited no detectable pro-apoptotic effects. Using the apoptosis-specific ELISA assay, we further validated that treatment of PHK with this dose of anti-sEcad had no effects on apoptosis. Collectively, this data shows that anti-sEcad treatment selectively induces apoptosis in two different human skin cancer cell lines, but not in normal primary human keratinocyte skin cultures.

### Example 3: Mouse SCCs are susceptible to anti-sEcad-induced apoptosis.

To effectively research a disease process such as squamous cell cancer, it is prudent to use both human and animal model approaches. Therefore, we wanted to determine whether anti-sEcad treatment would selectively induce apoptosis in a SCC mouse cell line. If so, this would enable further tests of the efficacy and toxicity of anti-sEcad agents *in vivo* using a murine model system.

We first determined, by ELISA, the levels of sEcad in the conditioned media of confluent primary mouse keratinocytes (PMK) and a mouse SCC cell line (PAM212). As expected, the level of sEcad in the PAM212 cell line was nearly two-fold higher than control PMK cultures. These levels were confirmed by western blotting; relative levels of sEcad were two to three-fold higher in the PAM212 cell line. Thus, it is clear that, as with breast and human skin cancer cells, the constitutive shedding of the ectodomain of E-cadherin is statistically higher in mouse SCCs than in non-cancerous healthy epithelial cells.

To validate that anti-sEcad treatment is selectively cytotoxic to mouse SCCs, PMK and PAM212 cells were cultured to confluency in the presence or absence of an antibody directed against the ectodomain of E-cadherin (20 µg/mL DECMA; Sigma) or pre-immune serum (IgG) for 48 hours, and analyzed for apoptosis using the TUNEL assay. We observed a marked increase in TUNEL-positive cells in the SCC cell line after incubation with the blocking antibody, but no appreciable difference in the PMK cultures.

These results were confirmed by an apoptosis-specific ELISA assay (the Cell Death Detection ELISA^{PLUS} (Boehringer Mannheim)) that showed over a 2-fold increase in apoptosis in the anti-sEcad treated PAM212 cell line and no appreciable cell death in PMK cultures. In contrast, levels of necrosis were unchanged in all the conditions tested irrespective of the cell type.

Since we previously demonstrated an increase in p53 levels in MCF-7 cells exposed to 20 µg/mL of anti-sEcad antibody, we next determined whether a similar increase in p53 would be present in PAM212 cells cultured in the presence or absence of this blocking antibody or pre-immune serum (IgG) for 24 and 48 hours. Briefly, 50 µg of lysates were electrophoresed on 4-15% gradient gels (BioRad), transferred to polyvinylidene difluoride (PVDF) membranes, and immunoblotted with anti-p53 (Santa Cruz; 1:400) followed by peroxidase-conjugated secondary antibody (1:3,000) and enhanced chemiluminescence (ECL) detection. Equal loading of protein was verified by G3PDH staining. Western blotting demonstrates a notable increase in p53 levels by 24 and 48 hours after anti-sEcad treatment, but no bands in untreated or isotype IgG controls. This timing of anti-sEcad induced p53 expression is the same as that found in the MCF-7 cell line, previously described. Therefore, it is likely that this anti-sEcad-induced cancer cell death is via a p53-dependent pathway, although the exact downstream players have yet to be elucidated.

### Example 4: Blocking sEcad does not induce apoptosis in other non-cancerous cells.

3T3 mouse fibroblasts and human endothelial cells (HUVEC) were cultured in the presence or absence of anti-sEcad Ab or pre-immune serum (IgG) for 24-48 hours and analyzed for apoptosis by TUNEL and by the apoptosis-specific ELISA assay described above. In the presence of the blocking antibody, 3T3 cells exhibited little to no apoptotic nuclei using the TUNEL assay and no change in apoptosis by the apoptosis-specific ELISA assay. Similarly, HUVEC cells treated with anti-sEcad exhibited no appreciable difference in apoptosis by using both strategies. Moreover, 3T3 and HUVEC cells exhibited no change in necrosis after culturing these cells in the E-cadherin blocking antibody.

### Example 5: Anti-sEcad mAb therapy delays tumor onset, prevents tumor burden, and lessens tumor grade in vivo.

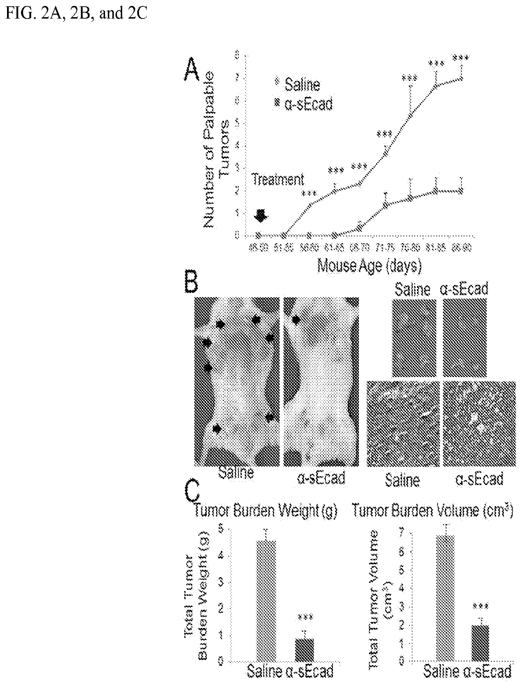

Female virgin MMTV-PyMT transgenic mice, characterized by rapid development of palpable breast cancer tumors that progress to aggressive adenocarcinomas with metastasis to the lungs, were used in this study (Guy *et al.,* 1992; Bugge *et al.,* 1998). Starting at 48 days of age, the mice were treated twice weekly with a monoclonal antibody targeting the EC-5 domain of sEcad (a-sEcad; DECMA-1, Sigma Aldrich, 20 µg/200 µl saline per mouse) or normal saline by i.p. injections. The mice were sacrificed at 90 days of age. All saline treated (control) MMTV-PyMT mice developed palpable tumors by 56-60 days whereas a statistically significant delay in tumor onset was observed in the treated mice (81-85 days; *see* FIG. 2A). 90-day old EC5 mAb-treated mice exhibited fewer total tumors that histopathologically were determined to be moderately differentiated (Architectural Grade of II and nuclear Grade of I; *see* FIG. 2B). In contrast, all tumors in 90 day saline treated mice were poorly differentiated (Architectural Grade of III and nuclear grade of III; *see* FIG. 2C). mAb-treated mice also displayed reduced total tumor weight and volume burden.

In conclusion, our results clearly demonstrate that anti-sEcad antibody treatment induces cell death in a variety of epithelial cancer cell lines (breast, skin and lung), while sparing adjacent normal healthy epithelial cells, fibroblasts and endothelial cells. We propose that cancer cells secrete sEcad in the microenvironment to artificially mimic normal cell-cell contacts and to provide a functional scaffolding with adjacent neighboring cells. Thus, by scavenging sEcad from the tumor microenvironment, we perturb this nurturing capacity of the tumor cell mileu and activate p53-dependent molecular pathways involved in programmed cell death.

A number of embodiments of the invention have been described.

## Claims

1. An antibody that specifically targets one or more of the second, third, fourth, or fifth subdomains (EC2, EC3, EC4 and EC5, respectively) of soluble E-cadherin (sEcad) but not the first subdomain (EC1) of sEcad for use in a method for the treatment of cancer.

2. An antibody for use in a method according to claim 1, wherein the antibody specifically targets EC4 and/or EC5.

3. An antibody for use in a method according to claim 1, wherein the antibody specifically targets EC2 and/or EC3.

4. An antibody for use in a method according to claim 1, wherein the cancer cells are epithelial-derived tumor cells.

5. An antibody for use in a method according to claim 1, wherein the antibody is a humanized, chimeric, single chain, or human antibody.

6. An antibody for use in a method according to claim 1, wherein the antibody is delivered in a pharmaceutical formulation that:
(a) produces, upon administration to a patient, a serum level of the antibody of 1-10 mg/kg, or,
(b) produces, upon addition to a cell culture, a concentration of the antibody of 1-500 µg/mL of cell culture medium.

7. An antibody for use in a method according to claim 1, wherein the antibody is delivered in a pharmaceutical formulation by oral administration, intravenous administration, nasal or inhalation administration, intramuscular administration, intraperitoneal administration, transmucosal administration, or transdermal administration.

8. An antibody for use in a method according to claim 1, wherein said antibody is administered together with a second cancer treatment.

9. An antibody for use in a method according to claim 1, wherein the cancer is a cancer of the alimentary canal, central nervous system, breast, skin, reproductive system, lung, or urinary tract.

10. An antibody for use in a method according to claim 1, wherein the antibody is detectably labeled.

11. An antibody for use in a method according to claim 1, wherein the cancer is squamous cell carcinoma or melanoma.

12. An antibody for use in a method according to claim 9, wherein the cancer of the alimentary canal is cancer of the mouth, throat, esophagus, stomach, intestine, rectum or anus.

13. An antibody for use in a method according to claim 9, wherein the cancer of the reproductive system is cervical cancer, uterine cancer, ovarian cancer, vulval or labial cancer, prostate cancer, testicular cancer, or cancer of the male genital tract.

14. A pharmaceutical formulation for use in a method for the treatment of cancer comprising the antibody of any one of claims 1-12, wherein the pharmaceutical formulation is free of a cytotoxic amount of an excipient.

15. The pharmaceutical formulation for use of claim 14, wherein the pharmaceutical formulation is formulated for delivery to a patient by intravenous administration.

## Patentansprüche

1. Antikörper, der spezifisch auf eine oder mehrere der zweiten, dritten, vierten oder fünften Subdomänen (EC2, EC3, EC4 bzw. EC5) von löslichem E-Cadherin (sEcad) zielt, nicht jedoch auf die erste Subdomäne (EC1) von sEcad, für die Verwendung in einem Verfahren zur Behandlung von Krebs.

2. Antikörper zur Verwendung in einem Verfahren nach Anspruch 1, wobei der Antikörper spezifisch auf EC4 und/oder EC5 zielt.

3. Antikörper zur Verwendung in einem Verfahren nach Anspruch 1, wobei der Antikörper spezifisch auf EC2 und/oder EC3 zielt.

4. Antikörper zur Verwendung in einem Verfahren nach Anspruch 1, wobei die Krebszellen epithelial derivierte Tumorzellen sind.

5. Antikörper zur Verwendung in einem Verfahren nach Anspruch 1, wobei der Antikörper ein humanisierter, chimärer, Einketten- oder Humanantikörper ist.

6. Antikörper zur Verwendung in einem Verfahren nach Anspruch 1, wobei der Antikörper in einer pharmazeutischen Zubereitung zugeführt wird, die:
(a) bei Verabreichung an einen Patienten einen Serumspiegel des Antikörpers von 1 bis 10 mg/kg erzeugt, oder
(b) bei Zugabe zu einer Zellkultur eine Konzentration des Antikörpers von 1 bis 500 µg/ml Zellkulturmedium erzeugt.

7. Antikörper zur Verwendung in einem Verfahren nach Anspruch 1, wobei der Antikörper in einer pharmazeutischen Zubereitung durch orale Verabreichung, intravenöse Verabreichung, nasale oder Inhalationsverabreichung, intramuskuläre Verabreichung, intraperitoneale Verabreichung, transmukosale Verabreichung oder transdermale Verabreichung zugeführt wird.

8. Antikörper zur Verwendung in einem Verfahren nach Anspruch 1, wobei der Antikörper gemeinsam mit einer zweiten Krebsbehandlung verabreicht wird.

9. Antikörper zur Verwendung in einem Verfahren nach Anspruch 1, wobei der Krebs ein Krebs des Verdauungstrakts, zentralen Nervensystems, der Brust, Haut, des reproduktiven Systems, der Lunge oder des Harnweges ist.

10. Antikörper zur Verwendung in einem Verfahren nach Anspruch 1, wobei der Antikörper detektierbar markiert ist.

11. Antikörper zur Verwendung in einem Verfahren nach Anspruch 1, wobei der Krebs ein Plattenepithelkarzinom oder Melanom ist.

12. Antikörper zur Verwendung in einem Verfahren nach Anspruch 9, wobei der Krebs des Verdauungstrakts ein Krebs des Mundes, Kehlkopfes, Ösophagus, Magens, Darms, Rektums oder Anus ist.

13. Antikörper zur Verwendung in einem Verfahren nach Anspruch 9, wobei der Krebs des reproduktiven Systems ein Krebs ein Zervixkarzinom, Gebärmutterkrebs, Ovarialkarzinom, Vulvakarzinom oder labialer Krebs, Prostatakrebs, Hodenkrebs oder Krebs des männlichen genitalen Trakts ist.

14. Pharmazeutische Zubereitung zur Verwendung in einem Verfahren für die Behandlung von Krebs, umfassend den Antikörper nach einem der Ansprüche 1 bis 12, wobei die pharmazeutische Zubereitung frei ist von einer cytotoxischen Menge eines Exzipienten.

15. Pharmazeutische Zubereitung zur Verwendung nach Anspruch 14, wobei die pharmazeutische Zubereitung zur Zuführung an einen Patienten durch intravenöse Verabreichung zubereitet wird.

## Revendications

1. Anticorps qui cible spécifiquement un ou plusieurs des deuxième, troisième, quatrième ou cinquième sous-domaines (EC2, EC3, EC4 et EC5, respectivement) de l'E-cadhérine soluble (sEcad) mais pas le premier sous-domaine (EC1) de sEcad, pour l'utilisation dans un procédé pour le traitement d'un cancer.

2. Anticorps pour l'utilisation dans un procédé selon la revendication 1, lequel anticorps cible spécifiquement EC4 et/ou EC5.

3. Anticorps pour l'utilisation dans un procédé selon la revendication 1, lequel anticorps cible spécifiquement EC2 et/ou EC3.

4. Anticorps pour l'utilisation dans un procédé selon la revendication 1, dans lequel les cellules cancéreuses sont des cellules tumorales d'origine épithéliale.

5. Anticorps pour l'utilisation dans un procédé selon la revendication 1, lequel anticorps est un anticorps humanisé, chimérique, à chaîne unique ou humain.

6. Anticorps pour l'utilisation dans un procédé selon la revendication 1, lequel anticorps est délivré dans une formulation pharmaceutique qui:
(a) produit, après administration à un patient, un taux sérique de l'anticorps de 1 à 10mg/kg, ou,
(b) produit, après addition à une culture cellulaire, une concentration de l'anticorps de 1 à 500µg/ml de milieu de culture cellulaire.

7. Anticorps pour l'utilisation dans un procédé selon la revendication 1, lequel anticorps est délivré dans une formulation pharmaceutique par administration orale, administration intraveineuse, administration nasale ou par inhalation, administration intramusculaire, administration intrapéritonéale, administration transmuqueuse ou administration transdermique.

8. Anticorps pour l'utilisation dans un procédé selon la revendication 1, lequel anticorps est administré conjointement avec un second traitement anticancéreux.

9. Anticorps pour l'utilisation dans un procédé selon la revendication 1, dans lequel le cancer est un cancer du canal alimentaire, du système nerveux central, du sein, de la peau, du système reproducteur, du poumon ou des voies urinaires.

10. Anticorps pour l'utilisation dans un procédé selon la revendication 1, lequel anticorps est marqué de façon détectable.

11. Anticorps pour l'utilisation dans un procédé selon la revendication 1, dans lequel le cancer est un carcinome cellulaire squameux ou un mélanome.

12. Anticorps pour l'utilisation dans un procédé selon la revendication 9, dans lequel le cancer du canal alimentaire est un cancer de la bouche, de la gorge, de l'œsophage, de l'estomac, de l'intestin, du rectum ou de l'anus.

13. Anticorps pour l'utilisation dans un procédé selon la revendication 9, dans lequel le cancer du système reproducteur est un cancer du col de l'utérus, un cancer utérin, un cancer ovarien, un cancer vulvaire ou labial, un cancer de la prostate, un cancer testiculaire, ou un cancer de l'appareil génital masculin.

14. Formulation pharmaceutique pour l'utilisation dans un procédé pour le traitement d'un cancer comprenant l'anticorps selon l'une quelconque des revendications 1 à 12, laquelle formulation pharmaceutique est exempte d'une quantité cytotoxique d'un excipient.

15. Formulation pharmaceutique pour l'utilisation selon la revendication 14, laquelle formulation pharmaceutique est formulée pour la délivrance à un patient par administration intraveineuse.
